# EUROPEAN PATENT APPLICATION

(11) **EP 4 600 253 A1**
(43) Date of publication of application: **13.08.2025**
(21) Application number: 23874886.7
(22) Date of filing: 04.10.2023
(51) Int. Cl.: C07H 15/12, C07C 271/22, C07D 207/46, C07H 1/00

(54) **METHOD FOR PRODUCING COMPOUND HAVING TWO-BRANCHED N-ACETYL-D-GALACTOSAMINE STRUCTURE**

(30) Priority: 05.10.2022 JP 2022161149
(71) Applicant: Daiichi Sankyo Company, Limited, Tokyo 103-8426 (JP)
(72) Inventor: NAGASAWA, Hiroshi, Tokyo 103-8426 (JP); ABE, Narumi, Tokyo 103-8426 (JP); NISHI, Yoshio, Tokyo 103-8426 (JP); ABE, Yuzo, Tokyo 103-8426 (JP); KOBAYASHI, Takumi, Tokyo 103-8426 (JP); SAKANISHI, Kohei, Tokyo 103-8426 (JP)
(74) Representative: Marks & Clerk LLP
(86) International application number: PCT/JP2023/036159
(87) International publication number: WO 2024/075760

(57) **Abstract**

An object of the present invention relates to a novel method for producing a biantennary N-acetyl-D-galactosamine ligand-oligonucleotide conjugate, and is particularly to provide a novel method for producing an N-acetyl-D-galactosamine unit, and a novel intermediate. Studies have been carried out on the selective deprotection of a benzyl group using a palladium catalyst in the novel method for producing an N-acetyl-D-galactosamine unit, and an industrially advantageous novel production method that can achieve high quality and a high yield, and a novel intermediate have been found, leading to the completion of the invention.

## Description

### Technical Field

The present invention relates to a novel method for producing a biantennary N-acetyl-D-galactosamine ligand-oligonucleotide conjugate, and particularly relates to an industrially advantageous novel method for producing a biantennary N-acetyl-D-galactosamine unit, and a novel intermediate.

### Background Art

As a method for delivering oligonucleotides to hepatic parenchymal cells in the liver, a method using a nucleic acid drug conjugate form in which N-acetyl-D-galactosamine (also referred to as GalNAc) is bonded to an oligonucleotide via a linker is known (Patent Literature 1), and a novel biantennary N-acetyl-D-galactosamine unit (also referred to as a biantennary GalNAc unit) that enables delivery of any oligonucleotide to the liver and a method for producing the unit using an Fmoc group, etc. as a protecting group have also been reported (Patent Literature 2). There is a demand for the development of a novel production method that can synthesize a large amount of a high-quality biantennary GalNAc unit, but it is generally extremely difficult to produce a compound such as a biantennary GalNAc unit that has a complex structure and many reactive sites. In particular, it is difficult to selectively introduce and remove a substituent and a protecting group during the production process to obtain a compound having a complex structure and many reactive sites with high quality and a high yield. For example, in a compound that includes both a trityl group and a benzyl group, an attempt has been made to selectively remove only the benzyl group, but the yield is about 35% (Non Patent Literature 1).

### Citation List

### Patent Literature

Patent Literature 1: International Publication No. WO 2019/172286
Patent Literature 2: International Publication No. WO 2021/049504

### Non Patent Literature

Non Patent Literature 1: J. Chem. Soc., Perkin Trans. 1, 1987, (3), 537-45

### Summary of Invention

### Technical Problem

An object of the present invention is to provide a method for producing an N-acetyl-D-galactosamine ligand-oligonucleotide conjugate, including a novel step for selectively introducing and removing a substituent and/or a protecting group.

### Solution to Problem

The present invention relates to a novel method for producing an N-acetyl-D-galactosamine ligand-oligonucleotide conjugate for solving the above problem, and particularly relates to a novel method for producing an N-acetyl-D-galactosamine unit, and a novel intermediate. In particular, the present invention relates to an industrially advantageous novel production method that can achieve high quality and a high yield, in which selective deprotection of a benzyl group using a palladium catalyst is a key reaction.

The present invention includes the following aspects [1] to [14].
[1] A method for producing the compound represented by formula (1): 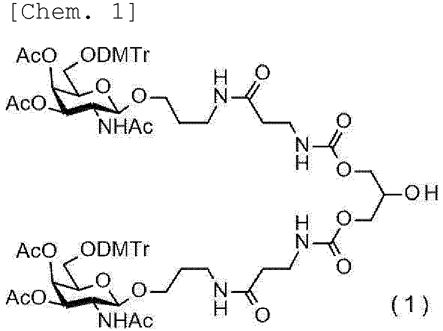
   wherein Ac represents an acetyl group, and DMTr represents a 4,4'-dimethoxytrityl group,
   the method comprising a step of reacting the compound represented by formula (2): 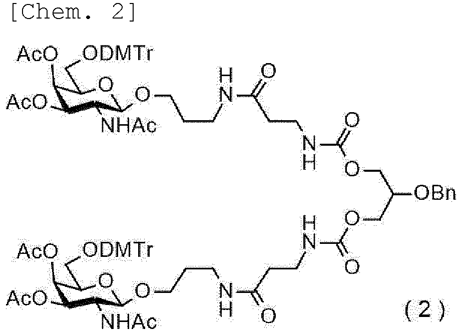
   wherein Ac represents an acetyl group, DMTr represents a 4,4'-dimethoxytrityl group, and Bn represents a benzyl group,
   with a palladium catalyst in the presence of a reducing agent in a reaction solvent.
[2] The production method according to [1], wherein the palladium catalyst is palladium on carbon or palladium hydroxide.
[3] The production method according to [1] or [2], wherein the reaction solvent is one or more solvents selected from an alcohol-based solvent, an ester-based solvent, an ether-based solvent, and an amide-based solvent.
[4] The production method according to [1] or [2], wherein the reaction solvent is one or more solvents selected from 2-propanol, dimethylacetamide, N-methyl-2-pyrrolidone, and 1,3-dimethyl-2-imidazolidinone.
[5] The production method according to [1] or [2], wherein the reaction solvent is a mixed solvent of ethyl acetate and one or more solvents selected from dimethylacetamide, N-methyl-2-pyrrolidone, and 1,3-dimethyl-2-imidazolidinone.
[6] A method for producing the compound represented by formula (3): 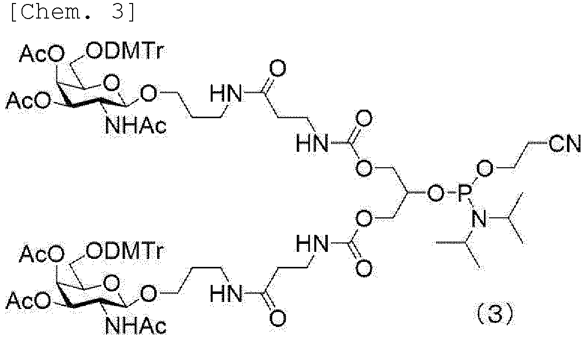
   wherein Ac represents an acetyl group, and DMTr represents a 4,4'-dimethoxytrityl group,
   or a salt thereof,
   the method comprising a step of reacting the compound represented by formula (1) obtained by the production method according to any one of [1] to [5] with an amidite reagent.
[7] A method for producing a conjugate consisting of an oligonucleotide and a biantennary N-acetyl-D-galactosamine unit, the biantennary N-acetyl-D-galactosamine unit being bonded to a 5' end or a 3' end of the oligonucleotide, the conjugate being represented by formula (4): 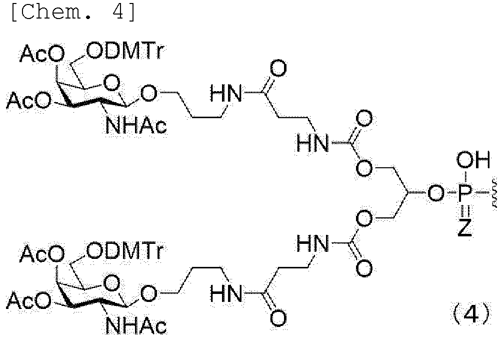
   wherein Ac represents an acetyl group, and DMTr represents a 4,4'-dimethoxytrityl group, and wherein Z represents an oxygen atom or a sulfur atom, and the bond at the right side of the structural formula represents a bond to the oligonucleotide,
   the method comprising a step of reacting the compound represented by formula (3) obtained by the production method according to [6] with the oligonucleotide.
[8] A method for producing the compound represented by formula (2) used in (1), comprising:
   a step of bonding the compound represented by formula (5): 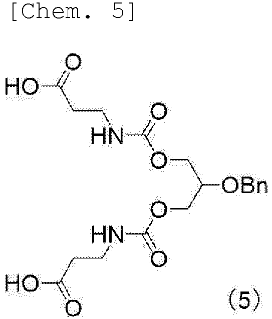
   wherein Bn represents a benzyl group,
   or a salt thereof, and
   the compound represented by formula (6): 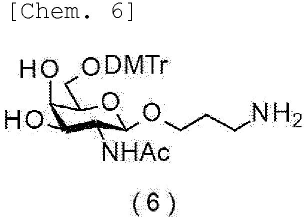
   wherein Ac represents an acetyl group, and DMTr represents a 4,4'-dimethoxytrityl group,
   by using a condensing agent; and
   a step of acetylating hydroxyl groups on the pyran ring by using an acetylation reagent.
[9] A method for producing the compound represented by formula (5) or a salt thereof used in [8], comprising:
   a step of reacting the compound represented by formula (7): 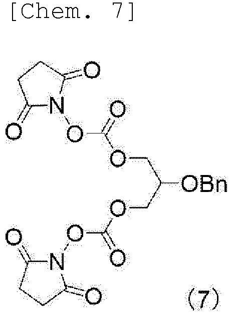
   wherein Bn represents a benzyl group,
   with β-alanine or a salt thereof, or
   a step of reacting the compound represented by formula (7) with a β-alanine alkyl ester or a salt thereof, wherein
   when the method comprises a step using a β-alanine alkyl ester or a salt thereof, the method further comprises a hydrolysis step.
[10] A method for producing the compound represented by formula (7) used in [9], comprising:
   a step of reacting 2-(benzyloxy)propane-1,3-diol with N,N'-disuccinimidyl carbonate in the presence of a base.
[11] A compound represented by formula (2): 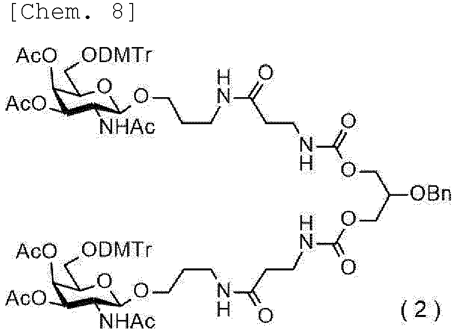 wherein Ac represents an acetyl group, DMTr represents a 4,4'-dimethoxytrityl group, and Bn represents a benzyl group.
[12] A compound represented by formula (8): 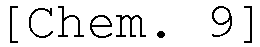 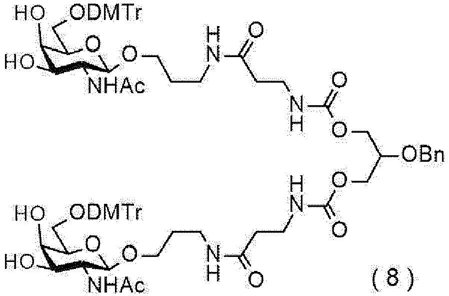 wherein Ac represents an acetyl group, DMTr represents a 4,4'-dimethoxytrityl group, and Bn represents a benzyl group.
[13] A compound represented by formula (5): 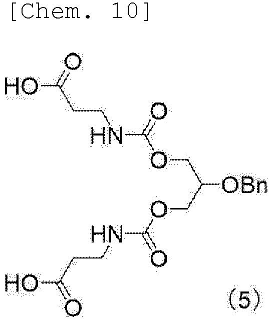
   wherein Bn represents a benzyl group,
   or a salt thereof.
[14] A compound represented by formula (7): 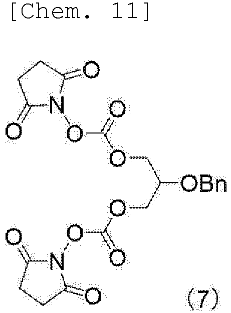 wherein Bn represents a benzyl group.
[15] A compound represented by formula (6): 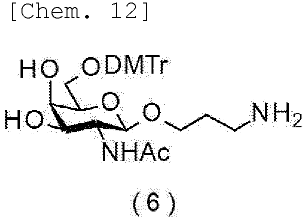
   wherein Ac represents an acetyl group, and DMTr represents a 4,4'-dimethoxytrityl group,
   or a salt thereof.

### Advantageous Effects of Invention

According to the present invention, it is possible to provide an industrially advantageous novel method for producing an N-acetyl-D-galactosamine unit with high yield and high quality, in which selective deprotection of a benzyl group using a palladium catalyst is a key reaction, and a novel intermediate. In particular, it is possible to provide a simple and inexpensive production method that does not use an expensive protecting group such as an Fmoc group. Thereby, it is possible to provide a novel method for producing an N-acetyl-D-galactosamine ligand-oligonucleotide conjugate.

### Description of Embodiments

Hereinafter, embodiments of the present invention are described in more detail.

The present invention provides a method for producing a conjugate represented by formula (4), consisting of an oligonucleotide and a biantennary N-acetyl-D-galactosamine unit, in which the biantennary N-acetyl-D-galactosamine unit is bonded to the **5'** end or the **3'** end of the oligonucleotide, by reacting the compound represented by formula (3) with the oligonucleotide. The present invention is also characterized by a process for obtaining the compound represented by formula (3), which is an intermediate.

That is, the present invention includes a method for producing the compound represented by formula (1) by using the compound represented by formula (2), and a method for producing the compound represented by formula (3) or a salt thereof by using the compound represented by formula (1) obtained above.

Furthermore, the present invention includes a method for reacting the compound represented by formula (5) or a salt thereof with the compound represented by formula (6) and further with the compound represented by formula (8) in order to produce the compound represented by formula (2), and a method for using the compound represented by formula (7) or compound (Va) in an Example described below in order to produce the compound represented by formula (5) or a salt thereof.

In addition, the present invention relates to intermediates obtained during the above methods.

The compounds represented by formulas (1) to (7) are sometimes referred to by different names herein. In addition, these names are sometimes listed together. The names of the compounds are shown in Table 1.

**[Table 1]**

| Compound | Another name | |
|---|---|---|
| Compound represented by formula (1) | Compound (1) | Compound (IXa) |
| Compound represented by formula (2) | Compound (2) | Compound (VIIIa) |
| Compound represented by formula (3) | Compound (3) | Compound (XIIa) |
| Compound represented by formula (4) | Compound (4) | - |
| Compound represented by formula (5) | Compound (5) | Compound (VIa) |
| Compound represented by formula (6) | Compound (6) | Compound (Ib) |
| Compound represented by formula (7) | Compound (7) | Compound (Va) |
| Compound represented by formula (8) | Compound (8) | Compound (VIIa) |

Hereinafter, synthesis processes according to one aspect of the present invention will be shown.

### Method for producing compound (Ib or 6)

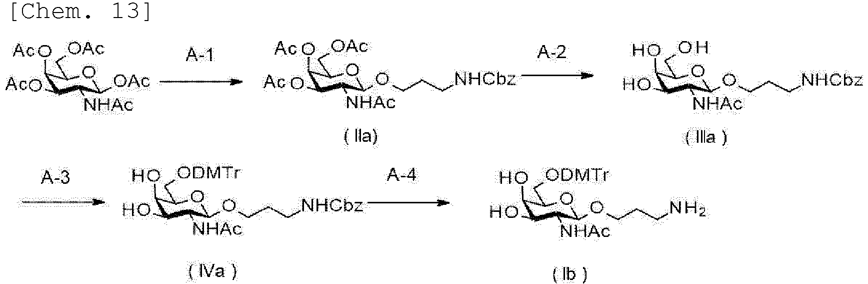

Step A-1: The present step is a step of producing compound (IIa) by carrying out a β-selective glycosylation reaction of 2-acetamido-1,3,4,6-tetra-O-acetyl-2-deoxy-β-D-galactopyranose with an amino alcohol by using an acid catalyst in an organic solvent. The solvent that can be used is not particularly limited as long as it is a solvent that does not inhibit the reaction, and examples thereof include one or more solvents selected from dichloromethane, acetonitrile, tetrahydrofuran, and the like. A preferable solvent is dichloromethane. The acid catalyst that can be used is not particularly limited as long as it is used for glycosylation, and examples thereof include trifluoromethanesulfonic acid, trimethylsilyl trifluoromethanesulfonate, and tin tetrachloride. A preferable acid catalyst is trifluoromethanesulfonic acid. The reaction temperature is usually 25°C to 45°C, and preferably 30°C to 40°C. The reaction time varies depending on the type and the amount of the acid catalyst used, and is usually 1 hour to 28 hours, and preferably 21 hours to 23 hours. The resulting compound can be purified by using a conventional method such as recrystallization or silica gel chromatography.

Step A-2: The present step is a step of producing compound (IIIa) by deprotecting acetyl (Ac) groups by reacting compound (IIa) with a deprotection reagent in an organic solvent. The deprotection reagent that can be used is not particularly limited as long as it is a reagent used for deprotecting acetyl groups, and examples thereof include an inorganic base such as sodium hydroxide, potassium hydroxide, or sodium carbonate, and an organic base such as ammonia, an alkylamine, sodium methoxide, or sodium ethoxide. A preferable deprotection reagent is sodium methoxide. The solvent that can be used is not particularly limited as long as it is a solvent that does not inhibit the reaction, and examples thereof include one or more solvents selected from methanol, ethanol, and the like. A preferable solvent is ethanol. The reaction temperature is usually 30°C to 50°C, and preferably 35°C to 45°C. The reaction time varies depending on the type and the amount of the deprotection reagent used, and is usually 0.5 hours to 24 hours, and preferably 12 hours to 14 hours. The resulting compound can be purified by using a conventional method such as recrystallization or silica gel chromatography.

Step A-3: The present step is a step of producing compound (IVa) by selectively protecting a primary hydroxyl group with a 4,4'-dimethoxytrityl (DMTr) group by reacting compound (IIIa) with a tritylation reagent such as 4,4'-dimethoxytrityl chloride or 4,4'-dimethoxytrityl trifluorosulfonate in an organic solvent. The solvent that can be used is not particularly limited as long as it is a solvent that does not inhibit the reaction, and examples thereof include N,N-dimethylacetamide, N,N-dimethylformamide, and N-methylpyrrolidone. A preferable solvent is N,N-dimethylacetamide. The reaction temperature is usually 15°C to 35°C, and preferably 20°C to 30°C. The reaction time varies depending on the type and the amount of the tritylation reagent used, and is usually 1 hour to 8 hours, and preferably 4 hours to 6 hours. The resulting compound can be purified by using a conventional method such as recrystallization or silica gel chromatography.

Step A-4: The present step is a step of producing compound (Ib or 6) by selectively deprotecting a benzyloxycarbonyl (Cbz) group of compound (IVa) by reacting compound (IVa) with a deprotection reagent in an organic solvent. The deprotection reagent that can be used is not particularly limited as long as it can deprotect a benzyloxycarbonyl group, and examples thereof include palladium on carbon or palladium hydroxide on carbon, and examples of the reducing agent include hydrogen, formic acid, or ammonium formate. A preferable metal catalyst is a palladium on carbon catalyst, and a preferable reducing agent is hydrogen. The solvent that can be used is not particularly limited as long as it is a solvent that does not inhibit the reaction, and examples thereof include one or more solvents selected from methanol, ethanol, tetrahydrofuran, and the like. A preferable solvent is methanol. The reaction temperature is usually 15°C to 35°C, and preferably 20°C to 30°C. When the reducing agent is hydrogen, the hydrogen pressure in the reaction system is usually 0.1 to 0.5 MPa, and preferably 0.2 to 0.4 MPa. The reaction time varies depending on the type and the amount of the deprotection reagent used, and is usually 0.5 hours to 6 hours, and preferably 2 hours to 3 hours. The resulting compound can be purified by using a conventional method such as recrystallization or silica gel chromatography.

### Method for producing compound (XIIa or 3)

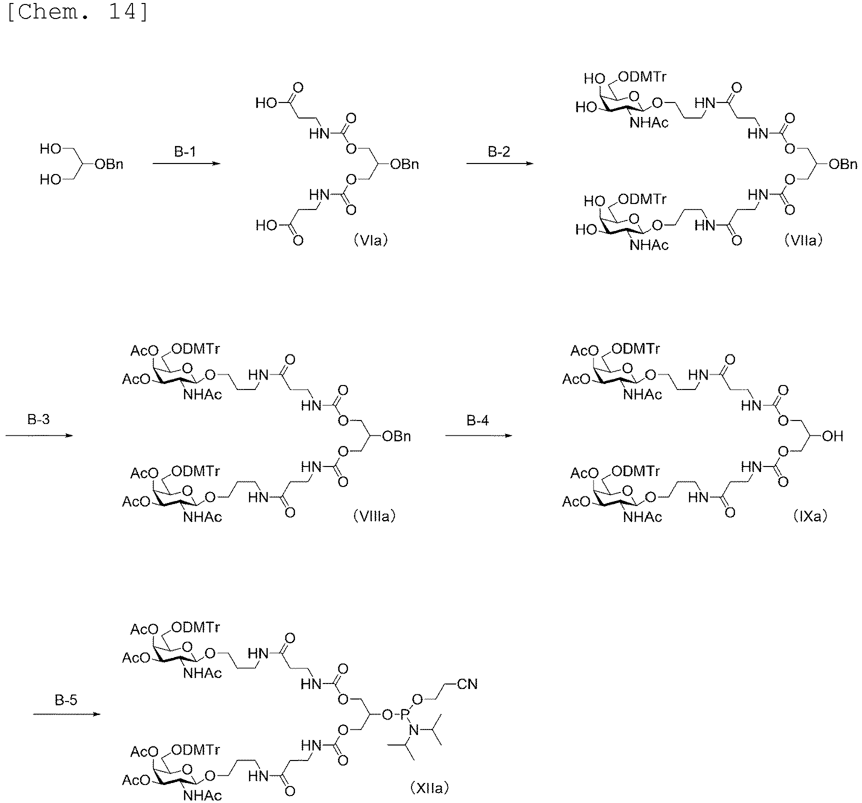

Step B-1: The present step includes a step of converting each of two hydroxyl groups of 2-(benzyloxy)propane-1,3-diol into an activated diester form (compound (Va or 7)) by reacting 2-(benzyloxy)propane-1,3-diol with N,N'-disuccinimidyl carbonate in an organic solvent (step B-1-1), and a step of producing compound (VIa or 5) by condensing compound (Va or 7) with β-alanine in the presence of a base (step B-1-2A), or a step of producing compound (VIa or 5) by condensing compound (Va or 7) with a β-alanine alkyl ester or a salt thereof in the presence of a base and then carrying out hydrolysis (step B-1-2B).

### (Step B-1-1)

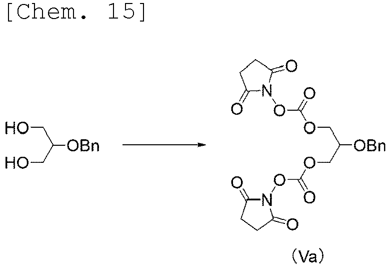

This is a step of converting 2-(benzyloxy)propane-1,3-diol into compound (Va or 7). Examples of a base that can be used include triethylamine and pyridine. A preferable base is pyridine. The solvent that can be used is not particularly limited as long as it is a solvent that does not inhibit the reaction, and examples thereof include acetonitrile, tetrahydrofuran, acetone, and N,N'-dimethylacetamide. A preferable solvent is acetonitrile. The reaction temperature is usually -15°C to 55°C, and preferably -5°C to 45°C. The reaction time is usually 1 hour to 6 hours, and preferably 2 hours to 3 hours.

### (Step B-1-2A)

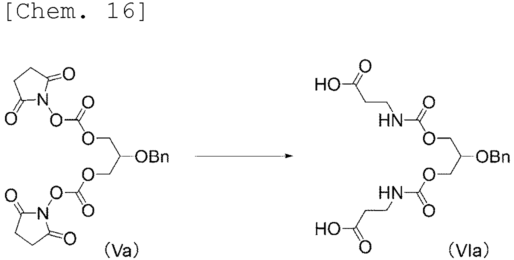

This is a step of producing compound (VIa or 5) by condensing β-alanine with compound (Va or 7) in the presence of a base. Examples of the base that can be used include triethylamine, pyridine, and N,N'-diisopropylethylamine. A preferable base is triethylamine. The solvent that can be used is not particularly limited as long as it is a solvent that does not inhibit the reaction, and examples thereof include aqueous acetonitrile, aqueous tetrahydrofuran, and aqueous acetone. A preferable solvent is aqueous acetonitrile. The reaction temperature is usually 15°C to 35°C, and preferably 20 to 30°C. The reaction time varies depending on the type and the amount of the base used, and is usually 0.25 hours to 6 hours, and preferably 0.5 hours to 1.5 hours. After completion of the reaction, compound (VIa or 5) can also be mixed with a base to form a salt. A preferable salt of compound (VIa or 5) is a dibenzylamine salt.

### (Step B-1-2B)

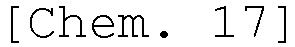

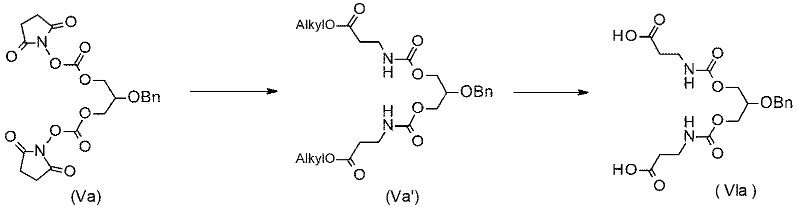

This is a step of producing compound (VIa or 7) by condensing a β-alanine alkyl ester with compound (Va or 7) in the presence of a base to obtain compound (Va'), wherein Alkyl represents a linear or branched C1-C6 alkyl group such as a methyl group, an ethyl group, a propyl group, an isopropyl group, or a tert-butyl group and then carrying out hydrolysis. Examples of the β-alanine alkyl ester that can be used include β-alanine methyl ester, β-alanine ethyl ester, β-alanine propyl ester, β-alanine isopropyl ester, β-alanine tert-butyl ester, and salts thereof. A preferable β-alanine alkyl ester is β-alanine ethyl ester or a salt thereof. Examples of the base that can be used in the condensation reaction include triethylamine, pyridine, and N,N'-diisopropylethylamine. A preferable base that can be used in the condensation reaction is triethylamine. The base that can be used in the hydrolysis reaction is not particularly limited as long as it is a base used in a normal hydrolysis reaction, and examples thereof include lithium hydroxide, sodium hydroxide, and potassium hydroxide. A preferable base that can be used in the hydrolysis reaction is potassium hydroxide. The solvent that can be used is not particularly limited as long as it is a solvent that does not inhibit the reaction, and examples thereof include acetonitrile, tetrahydrofuran, and acetone. A preferable solvent is tetrahydrofuran. The reaction temperature is usually 15°C to 35°C, and preferably 20 to 30°C. The reaction time varies depending on the type and the amount of the base used, and is usually 0.25 hours to 6 hours, and preferably 0.5 hours to 1.5 hours. Compound (VIa or 5) can also be mixed with a base to form a salt. A preferable salt of compound (VIa or 5) is a dibenzylamine salt.

Step B-2: The present step is a step of producing compound (VIIa or 8) by reacting compound (Ib or 6) with compound (VIa or 5) or a salt thereof by using a condensing agent in an organic solvent. When a salt of compound (VIa or 5) is used, the salt is changed to free acid by a separation procedure or the like before carrying out the reaction, and then used in the reaction. The condensing agent that can be used is not particularly limited as long as it is a reagent used in a condensation reaction between a carboxylic acid and an amino group, and examples thereof include a combination of 1-hydroxybenzotriazole monohydrate and 1-(3-dimethylaminopropyl)-3-ethylcarbodiimide hydrochloride, 4-(4,6-dimethoxy-1,3,5-triazin-2-yl)-4-methylmorphonium chloride n-hydrate, and O-(7-azabenzotriazol-1-yl)-N,N,N',N'-tetramethyluronium hexafluorophosphate. A preferable condensing agent is a combination of 1-hydroxybenzotriazole monohydrate and 1-(3-dimethylaminopropyl)-3-ethylcarbodiimide hydrochloride. The solvent that can be used is not particularly limited as long as it is a solvent that does not inhibit the reaction, and examples thereof include aqueous tetrahydrofuran, aqueous acetonitrile, and aqueous acetone. A preferable solvent is aqueous tetrahydrofuran. The reaction temperature is usually 10°C to 40°C, and preferably 20 to 30°C. The reaction time varies depending on the type and the amount of the condensing reagent used, and is usually 5 hours to 60 hours, and preferably 45 hours to 47 hours. The resulting compound can be purified by using a conventional method such as recrystallization or silica gel chromatography.

Step B-3: The present step is a step of producing compound (VIIIa or 2) by reacting compound (VIIa or 8) with an acetylation reagent in an organic solvent. The acetylation reagent that can be used is not particularly limited as long as it is a reagent used in an acetylation reaction, and examples thereof include acetic anhydride and acetyl chloride. A preferable acetylation reagent is acetic anhydride, and it is more preferable to use acetic anhydride in combination with 4-dimethylaminopyridine and triethylamine. The solvent that can be used is not particularly limited as long as it is a solvent that does not inhibit the reaction, and examples thereof include one or more solvents selected from ethyl acetate, tetrahydrofuran, acetone, acetonitrile, and the like. A preferable solvent is a mixed solvent of ethyl acetate and tetrahydrofuran. The reaction temperature is usually 10°C to 40°C, and preferably 20 to 30°C. The reaction time varies depending on the type and the amount of the acetylation reagent used, and is usually 2 hours to 12 hours, and preferably 7 hours to 9 hours. The compound (VIIIa or 2) obtained in the present step can be used in the next step without isolation and purification, and can also be purified by using a conventional method such as recrystallization or silica gel chromatography.

Step B-4: The present step is a step of producing compound (IXa or 1) by reacting compound (VIIIa or 2) with a deprotection reagent for a benzyl group in the presence of a reducing agent in an organic solvent to selectively deprotect the benzyl group. Examples of the deprotection reagent for a benzyl group include a combination of a palladium catalyst supported on a support and a reducing agent. The palladium catalyst that can be used is not particularly limited as long as it can deprotect a benzyl group, and preferable examples thereof include a palladium on carbon catalyst and a palladium hydroxide on carbon catalyst. The palladium content of the catalyst of a palladium on carbon catalyst and a palladium hydroxide on carbon catalyst is not particularly limited and, for example, a catalyst having a palladium content of 5%, 10%, 20%, or the like can be used. A palladium catalyst having a preferable content is a 5% palladium catalyst or a 20% palladium hydroxide catalyst. Examples of the reducing agent that can be used include hydrogen, formic acid, and ammonium formate. A preferable reducing agent is hydrogen.

The solvent that can be used in the present step is not particularly limited as long as it is a solvent that does not inhibit the reaction, and examples thereof include one or more solvents selected from an alcohol-based solvent, an ester-based solvent, an ether-based solvent, and an amide-based solvent. The solvent is, for example, one or more solvents selected from methanol, ethanol, 1-propanol, 2-propanol, ethyl acetate, propyl acetate, tetrahydrofuran, 2-methyltetrahydrofuran, tert-butyl methyl ether, cyclopentyl methyl ether, dimethylacetamide, dimethylformamide, N-methyl-2-pyrrolidone, 1,3-dimethyl-2-imidazolidinone, and the like. A preferable solvent is one or more solvents selected from 2-propanol, dimethylacetamide, N-methyl-2-pyrrolidone, and 1,3-dimethyl-2-imidazolidinone, or a mixed solvent of ethyl acetate and one or more solvents selected from dimethylacetamide, N-methyl-2-pyrrolidone, and 1,3-dimethyl-2-imidazolidinone. A more preferable solvent is a mixed solvent of ethyl acetate and one or more solvents selected from dimethylacetamide, N-methyl-2-pyrrolidone, and 1,3-dimethyl-2-imidazolidinone. The reaction temperature is usually 10°C to 40°C, and preferably 20 to 30°C. The reaction time varies depending on the types and the amounts of the palladium catalyst and the reducing agent used, and is usually 4 hours to 10 hours, and preferably 6 hours to 8 hours.

In the present step, "selectively deprotect the benzyl group" means that a 4,4'-dimethoxytrityl group is not deprotected and a compound in which only the benzyl group is deprotected is generated, and means that a compound in which only the benzyl group is deprotected is generated as the main product. For example, this means that a compound in which only the benzyl group is deprotected is generated with an HPLC area ratio of 40% or more, preferably 50% or more, and more preferably 60% or more.

Step B-5: The present step is a step of producing compound (VIIa or 3) by reacting compound (IXa or 1) with an activating agent and an amidite reagent in the presence of a drying agent in an organic solvent. The amidite reagent that can be used is not particularly limited as long as it is a reagent used to introduce a phosphorus atom-containing group useful for forming a covalent bond between a hydroxyl group of compound (IXa or 1) and an oligonucleotide, and examples thereof include 2-cyanoethyl N,N,N',N'-tetraisopropylphosphordiamidite and 2-cyanoethyl diisopropylchlorophosphoramidite. A preferable amidite reagent is 2-cyanoethyl N,N,N',N'-tetraisopropylphosphordiamidite. The amount of the amidite reagent that can be used is 1.0 to 1.6 equivalents and preferably 1.2 to 1.4 equivalents based on compound (IXa or 1). The activating agent that can be used is not particularly limited as long as it is a reagent that can be used to form an active intermediate in the amidite formation, and examples thereof include 5-benzylthiotetrazole, 5-phenyltetrazole, dibromoimidazole, 4,5-dicyanoimidazole, and an N-alkylimidazole trifluoroacetate. A preferable activating agent is 4,5-dicyanoimidazole. The amount of the activating agent that can be used is 0.1 to 1.0 equivalents and preferably 0.4 to 0.6 equivalents based on compound (IXa or 1). The drying agent that can be used is not particularly limited as long as it is a reagent used to absorb moisture in the reaction solution, and examples thereof include molecular sieve 3A, molecular sieve 4A, and molecular sieve 5A. A preferable drying agent is molecular sieve 4A. The solvent that can be used is not particularly limited as long as it is a solvent that does not inhibit the reaction, and examples thereof include one or more solvents selected from ethyl acetate, dichloromethane, N,N-dimethylformamide, tetrahydrofuran, 2-methyltetrahydrofuran, methyl isobutyl ketone, and the like. A preferable solvent is a mixed solvent of ethyl acetate and dichloromethane. The reaction temperature is usually -5°C to 10°C, and preferably 0°C to 5°C. The reaction time varies depending on the types and the amounts of the reagents used, and when the preferable activating agent, amidite reagent, and drying agent are used, the reaction time is usually 12 hours to 24 hours, and preferably 15 hours to 17 hours.

### Method for producing compound represented by formula (4)

A conjugate represented by formula (4), consisting of an oligonucleotide and a biantennary N-acetyl-D-galactosamine unit, in which the biantennary N-acetyl-D-galactosamine unit is bonded to the 5' end or the 3' end of the oligonucleotide, includes a step of reacting the compound (XIIa or 3) produced by the above production method with the oligonucleotide.

Hereinafter, each step of the above processes is described in detail.

In the present invention, the "palladium catalyst" means a palladium catalyst used to eliminate a benzyl group bonded to a hydroxyl group, and is preferably palladium on carbon or palladium hydroxide on carbon.

In the present invention, examples of the "reducing agent" used in an elimination reaction of a benzyl group using a palladium catalyst include hydrogen, formic acid, and ammonium formate. The reducing agent is preferably hydrogen.

As the "reaction solvent" used in the present invention, any solvent that does not inhibit the reaction can be used. Examples of the solvent that can be used in an elimination reaction of a benzyl group using a palladium catalyst include an alcohol-based solvent, an ester-based solvent, an ether-based solvent, and amide-based solvent.

Examples of the alcohol-based solvent that can be used in the benzyl group elimination step using a palladium catalyst according to the present invention include methanol, ethanol, 1-propanol, and 2-propanol. The alcohol-based solvent is preferably 2-propanol.

Examples of the ester-based solvent that can be used in the benzyl group elimination step using a palladium catalyst according to the present invention include ethyl acetate and propyl acetate. The ester-based solvent is preferably ethyl acetate.

Examples of the ether-based solvent that can be used in the benzyl group elimination step using a palladium catalyst according to the present invention include tetrahydrofuran, 2-methyltetrahydrofuran, tert-butyl methyl ether, and cyclopentyl methyl ether. The ether-based solvent is preferably 2-methyltetrahydrofuran.

Examples of the amide-based solvent that can be used in the benzyl group elimination step using a palladium catalyst according to the present invention include dimethylacetamide, dimethylformamide, N-methyl-2-pyrrolidone, and 1,3-dimethyl-2-imidazolidinone. The amide-based solvent is preferably dimethylacetamide, N-methyl-2-pyrrolidone, or 1,3-dimethyl-2-imidazolidinone.

A preferable solvent that can be used in the benzyl group elimination step using a palladium catalyst according to the present invention is one or more solvents selected from methanol, 2-propanol, ethyl acetate, 2-methyltetrahydrofuran, dimethylacetamide, N-methyl-2-pyrrolidone, and 1,3-dimethyl-2-imidazolidinone. A more preferable solvent is one or more solvents selected from 2-propanol, dimethylacetamide, N-methyl-2-pyrrolidone, and 1,3-dimethyl-2-imidazolidinone.

In the benzyl group elimination step using a palladium catalyst according to the present invention, a mixed solvent of two or more solvents is preferably a mixed solvent of an amide-based solvent and ethyl acetate, and more preferably a mixed solvent of one or more solvents selected from dimethylacetamide, N-methyl-2-pyrrolidone, or 1,3-dimethyl-2-imidazolidinone, and ethyl acetate. The mixed solvent is most preferably a mixed solvent of N-methyl-2-pyrrolidone and ethyl acetate.

When a mixed solvent of N-methyl-2-pyrrolidone and ethyl acetate is used, these can be mixed at any ratio and used. The ratio of N-methyl-2-pyrrolidone:ethyl acetate is preferably 1:30 to 1:1, and more preferably 1:5 to 1:2.

In the present invention, the "amidite reagent" is a reagent used in the amidite formation step, and is used to introduce a phosphorus atom-containing group useful for forming a covalent bond between a hydroxyl group of a biantennary N-acetyl-D-galactosamine unit and an oligonucleotide. Examples of the amidite reagent include 2-cyanoethyl N,N,N',N'-tetraisopropylphosphordiamidite and 2-cyanoethyl diisopropylchlorophosphoramidite. The amidite reagent is preferably 2-cyanoethyl N,N,N',N'-tetraisopropylphosphordiamidite.

The amidite formation step in the present invention is a step of producing the compound represented by general formula (3) by reacting the compound represented by formula (1) with an activating agent and an amidite reagent in the presence of a drying agent in an organic solvent.

The solvent that can be used in the amidite formation step is, for example, one or more solvents selected from ethyl acetate, dichloromethane, N,N-dimethylformamide, tetrahydrofuran, 2-methyltetrahydrofuran, and methyl isobutyl ketone, and is preferably a mixed solvent of ethyl acetate and dichloromethane.

The activating agent that can be used in the amidite formation step is a reagent used to form an active intermediate of the amidite reagent, and examples thereof include 5-benzylthiotetrazole, 5-phenyltetrazole, dibromoimidazole, 4,5-dicyanoimidazole, and an N-alkylimidazole trifluoroacetate. The activating agent is preferably 4,5-dicyanoimidazole.

The drying agent that can be used in the amidite formation step is a reagent used to absorb moisture in the reaction solution, and examples thereof include molecular sieve 3A, molecular sieve 4A, and molecular sieve 5A. The drying agent is preferably molecular sieve 4A.

By bonding the amidite form of the biantennary N-acetyl-D-galactosamine unit represented by formula (3) produced by the production method of the present invention and an oligonucleotide having a desired nucleotide sequence by using a conventional method such as a phosphoramidite method, a conjugate form consisting of the biantennary N-acetyl-D-galactosamine unit represented by formula (4) and the oligonucleotide can be produced. As used herein, the terms "conjugate" and "conjugate form" each refer to a compound in which an N-acetyl-D-galactosamine unit (GalNAc unit) is bonded to the 5' end and/or the 3' end of an oligonucleotide. The production thereof can be carried out by using a DNA synthesizer, such as PerkinElmer's Model 392 using the phosphoramidite method, according to the method disclosed in Nucleic Acids Research, 12, 4539 (1984), or the like. More specifically, a biantennary N-acetyl-D-galactosamine ligand-oligonucleotide conjugate form can be produced by the production method disclosed in Patent Literature 2 (International Publication No. WO 2021/049504).

The method for producing the oligonucleotide used in the present invention is not particularly limited as long as the desired oligonucleotide can be synthesized, and a known chemical synthesis method (such as a phosphotriester method, a phosphoramidite method, or a H-phosphonate method) can be used. For example, the oligonucleotide can be synthesized by using a commercially available nucleic acid synthesizer and using a commercially available reagent used for DNA/RNA synthesis.

The oligonucleotide having a desired nucleotide sequence can be synthesized by the conventional phosphoramidite method by using a DNA synthesizer such as PerkinElmer's Model 392 using the phosphoramidite method, according to the method disclosed in Nucleic Acids Research, 12, 4539 (1984), or the like.

As amidite reagents corresponding to various nucleosides, those commercially available may be purchased and used, or such amidite reagents can also be synthesized according to known methods.

In the present invention, examples of the "acetylation reagent" include acetic anhydride and acetyl chloride. The acetylation reagent is preferably acetic anhydride.

As used herein, the "condensing agent" means a reagent used in a condensation reaction between a carboxylic acid and an amino group, and examples thereof include a combination of 1-hydroxybenzotriazole monohydrate and 1-(3-dimethylaminopropyl)-3-ethylcarbodiimide hydrochloride, 4-(4,6-dimethoxy-1,3,5-triazin-2-yl)-4-methylmorphonium chloride n-hydrate, and O-(7-azabenzotriazol-1-yl)-N,N,N',N'-tetramethyluronium hexafluorophosphate. The condensing agent is preferably a combination of 1-hydroxybenzotriazole monohydrate and 1-(3-dimethylaminopropyl)-3-ethylcarbodiimide hydrochloride.

In the step of condensation with the compound represented by formula (7) according to the present invention, β-alanine, a β-alanine alkyl ester, or a salt thereof can be used. Examples of the β-alanine alkyl ester include β-alanine methyl ester, β-alanine ethyl ester, β-alanine propyl ester, β-alanine isopropyl ester, β-alanine tert-butyl ester, and salts thereof. In the step of condensation, β-alanine or a salt thereof, or β-alanine ethyl ester or a salt thereof, is preferable.

Examples of the base that can be used in the step of condensation with the compound represented by formula (7) according to the present invention include triethylamine, pyridine, and N,N'-diisopropylethylamine. The base is preferably triethylamine.

The compound represented by formula (1) according to the present invention, the compound represented by formula (2) according to the present invention, the compound represented by formula (3) according to the present invention, the compound represented by formula (4) according to the present invention, the compound represented by formula (6) according to the present invention or a salt thereof, and the compound represented by formula (8) according to the present invention include all isomers (a diastereoisomer, an optical isomer, and a geometric isomer) thereof.

The compound represented by formula (3) according to the present invention, the compound represented by formula (4) according to the present invention, the compound represented by formula (5) according to the present invention, and the compound represented by formula (6) according to the present invention can each be converted into a salt by reacting with a base if these have an acidic group, and can be converted into a salt by reacting with an acid if these have a basic group.

Examples of the salt based on a basic group include a hydrohalide such as a hydrofluoride, a hydrochloride, a hydrobromide, or a hydroiodide; an inorganic acid salt such as a nitrate, a perchlorate, a sulfate, or a phosphate; an alkylsulfonate such as a methanesulfonate, a trifluoromethanesulfonate, and an ethanesulfonate; an arylsulfonate such as a benzenesulfonate or a p-toluenesulfonate; an organic acid salt such as an acetate, a malate, a fumarate, a succinate, a citrate, an ascorbate, a tartrate, an oxalate, or an adipate; and an amino acid salt such as a glycine salt, a lysine salt, an arginine salt, an ornithine salt, a glutamate, or an aspartate.

Examples of the salt based on an acidic group include an alkali metal salt such as a sodium salt, a potassium salt, or a lithium salt; an alkaline earth metal salt such as a calcium salt or a magnesium salt; a metal salt such as an aluminum salt or an iron salt; an inorganic salt such as an ammonium salt; an amine salt such as an organic salt such as a tert-octylamine salt, a dibenzylamine salt, a morpholine salt, a glucosamine salt, a phenylglycine alkyl ester salt, an ethylenediamine salt, an N-methylglucamine salt, a guanidine salt, a diethylamine salt, a triethylamine salt, a dicyclohexylamine salt, an N,N'-dibenzylethylenediamine salt, a chloroprocaine salt, a procaine salt, a diethanolamine salt, an N-benzylphenethylamine salt, a piperazine salt, a tetramethylammonium salt, or a tris(hydroxymethyl)aminomethane salt; and an amino acid salt such as a glycine salt, a lysine salt, an arginine salt, an ornithine salt, a glutamate, or an aspartate.

A preferable salt of the compound represented by formula (8) according to the present invention is a dibenzylamine salt.

The compound of the present invention or a salt thereof may incorporate a water molecule to form a hydrate when left in the air or recrystallized, and such a hydrate is also included in the compound of the present invention or a salt thereof.

The compound of the present invention or a salt thereof may absorb a certain solvent to form a solvate when left in the solvent or recrystallized, and such a solvate is also included in the compound of the present invention or a salt thereof.

As used herein, the "GalNAc unit" means a partial structure including N-acetyl-D-galactosamine (GalNAc) that can bind to an asialoglycoprotein receptor (ASGPR) on a hepatic parenchymal cell in the liver. The GalNAc unit may include a phosphate group or a thiophosphate group for binding between a linear or branched linker structure and an oligonucleotide. The number of GalNAcs included in one GalNAc unit is not limited, and unless otherwise specified, a known one, one disclosed herein, or the like can be adopted. The structure of GalNAc may be modified as long as the ability to bind to an ASGPR is maintained. In addition, GalNAc having a protecting group introduced during the production process, or the like, is also included.

In addition, as used herein, the "oligonucleotide" refers to a nucleic acid having a chain length of 100 bases or less that can be synthesized, and may be single-stranded or double-stranded. As the nucleic acid to be adopted, DNA, RNA, a natural nucleic acid, a modified nucleic acid, or the like can be selectively used depending on the purpose, as described later, and these may be mixed in one oligonucleotide. As the GalNAc unit and the oligonucleotide, for example, those disclosed in Patent Literature 2 (International Publication No. WO 2021/049504) may be used.

### Examples

Next, the present invention will be described. It should be understood that the reaction conditions of the present invention are not limited to those described herein. In the present invention, a functional group in a compound may be protected with an appropriate protecting group. Examples of such a functional group can include a hydroxyl group, a carboxy group, and an amino group, and the types of protecting groups and the conditions for introducing and removing the protecting groups can be determined with reference to those disclosed in, for example, Protective Groups in Organic Synthesis (T. W. Green and P. G. M. Wuts, John Wiley & Sons, Inc., New York, 2006).

The abbreviations used in the Examples have the following meanings.
g: grams, mL: milliliters, mol: mole, MHz: megahertz, Ac: acetyl group, DMTr: 4,4'-dimethoxytrityl group, Bn: benzyl group, Cbz: benzyloxycarbonyl group, MeOH: methanol, IPA: 2-propanol, EtOAc: ethyl acetate, 2-MeTHF: 2-methyltetrahydrofuran, DMAc: N,N-dimethylacetamide, NMP: N-methyl-2-pyrrolidone, DMI: 1,3-dimethyl-2-imidazolidinone, DMSO: dimethylsulfoxide

In the following Examples, nuclear magnetic resonance (hereinafter, ¹H NMR: 500 MHz) spectra were indicated by δ values (ppm) as chemical shift values determined by using chloroform-d, methanol-d4, dimethylsulfoxide-d6, and acetonitrile-d3 as deuterated solvents and using tetramethylsilane as the standard substance. Splitting patterns were designated as s for singlet, d for doublet, dd for double doublet, t for triplet, q for quartet, m for multiplet, and br for broad.

### (Example 1)

### Production of 3-aminopropyl 2-acetamido-6-O-[bis(4-methoxyphenyl)(phenyl)methyl]-2-deoxy-β-D-galactopyranoside (Ib or 6)

### (Example 1-1)

### Production of benzyl {3-[(2-acetamido-3,4,6-tri-O-acetyl-2-deoxy-β-D-galactopyranosyl)oxy]propyl}carbamate (IIa)

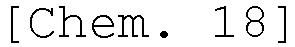

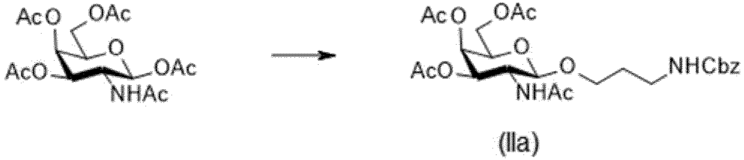

2-Acetamido-1,3,4,6-tetra-O-acetyl-2-deoxy-β-D-galactopyranose (60.0 g, 154.0 mmol) was added to dichloromethane (900 mL), and the resulting mixture was concentrated under reduced pressure to obtain a dichloromethane solution (600 mL). Similarly, dichloromethane (300 mL) was added to the obtained dichloromethane solution, and the resulting mixture was concentrated under reduced pressure to obtain a dichloromethane solution (600 mL). It was confirmed that the water content value of the concentrate of the obtained dichloromethane solution was 0.02% or less by using a Karl Fischer moisture meter (coulometric method). Dichloromethane (300 mL) was added, benzyl (3-hydroxypropyl)carbamate (33.9 g, 162.0 mmol) and trifluoromethanesulfonic acid (4.6 g, 30.8 mmol) were added in this order, and the resulting mixture was stirred at 35°C for 22 hours. After confirmation of termination of the reaction, the reaction solution was cooled to 25°C and separated with 8% aqueous sodium bicarbonate (180 mL). The resulting organic layer was concentrated under reduced pressure to 300 mL. Ethanol (900 mL) was added to the resulting concentrate, and the resulting mixture was then concentrated under reduced pressure to obtain 900 mL of a solution. Subsequently, the same procedure of concentration under reduced pressure was repeated again, and ethanol (300 mL) was added to the resulting solution (900 mL) to obtain a solution of the title compound (IIa) in ethanol (1200 mL).

The title compound (IIa) was synthesized (28.99 g, yield: 83.8%) by using 2-acetamido-1,3,4,6-tetra-O-acetyl-2-deoxy-β-D-galactopyranose (25.0 g, 64.21 mmol) in the same manner as in (Example 1-1). ¹H-NMR (500 MHz, CDCl₃): δ 7.40-7.31 (5H, m), 6.19 (1H, d, J = 8.5 Hz), 5.32 (1H, d, J = 2.0 Hz), 5.12 and 5.08 (2H, d, J = 12.0 Hz), 5.01-4.96 (2H, m), 4.33 (1H, d, J = 8.5 Hz), 4.18-4.09 (3H, m), 3.97 (1H, ddd, J = 10.0, 4.0, 4.0 Hz), 3.77 (1H, dd, J = 7.0, 7.0 Hz), 3.59-3.52 (1H, m), 3.40 (1H, ddd, J = 9.5, 9.5, 3.0 Hz), 3.12-3.07 (1H, m), 2.15 (3H, s), 2.05 (3H, s), 2.01 (3H, s), 1.94 (3H, s), 1.85-1.78 (1H, m), 1.66-1.58 (1H, m)

### (Example 1-2)

### Production of benzyl {3-[(2-acetamido-2-deoxy-β-D-galactopyranosyl)oxy]propyl}carbamate (IIIa)

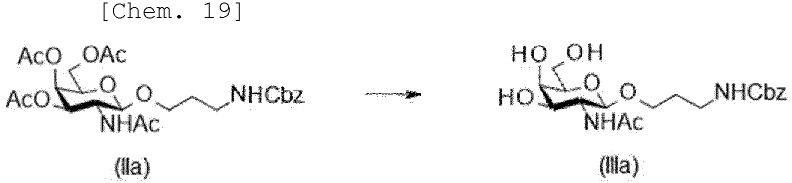

A solution of benzyl {3-[(2-acetamido-3,4,6-tri-O-acetyl-2-deoxy-β-D-galactopyranosyl)oxy]propyl}carbamate (IIa) in ethanol (1200 mL) was heated to 40°C, and a 28% sodium methoxide methanol solution (5.9 g, 30.8 mmol) was added, and then the resulting mixture was stirred for 13 hours. After confirmation of termination of the reaction, the reaction solution was cooled to 0°C, and the precipitated solid was filtered, washed with ethanol (180 mL) at 0°C, and dried under reduced pressure at an external temperature of 40°C to obtain the title compound (IIIa) (56.4 g, area ratio by liquid chromatography analysis: 99.8%, total yield for two steps: 88.7%). ¹H-NMR (500 MHz, DMSO-d6): δ 7.60 (1H, d, J = 9.5 Hz), 7.38-7.29 (5H, m), 7.19 (1H, dd, J = 6.0, 6.0 Hz), 5.01 (2H, s), 4.60-4.56 (2H, m), 4.49 (1H, d, J = 4.0 Hz), 4.21 (1H, d, J = 8.0 Hz), 3.74-3.67 (2H, m), 3.64 (1H, dd, J = 3.5, 3.5 Hz), 3.56-3.47 (2H, m), 3.42-3.32 (2H, m), 3.30 (1H, dd, J = 5.5, 5.5 Hz), 3.06-2.99 (2H, m), 1.81 (3H, s), 1.60 (2H, tt, J = 6.5, 6.5 Hz)

### (Example 1-3)

### Production of benzyl [3-({2-acetamido-6-0-[bis(4-methoxyphenyl)(phenyl)methyl]-2-deoxy-β-D-galactopyranosyl}oxy)propyl]carbamate (IVa)

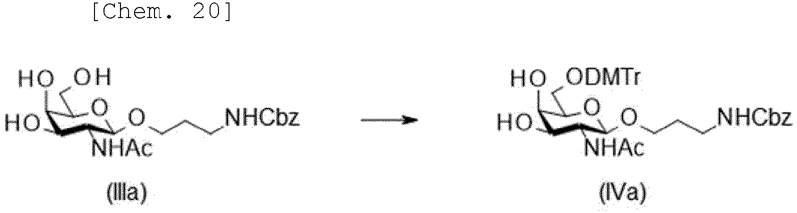

Benzyl {3-[(2-acetamido-2-deoxy-β-D-galactopyranosyl)oxy]propyl}carbamate (IIIa) (54.0 g, 131.0 mmol) and pyridine (31.1 g, 393.0 mmol) were added to N,N-dimethylacetamide (324 mL) in this order, and the resulting mixture was stirred. Subsequently, 4,4'-dimethoxytrityl chloride (26.6 g, 78.6 mmol) was added, and then rinsing with toluene (54 mL) was repeated twice, followed by stirring for 5 hours. After confirmation of termination of the reaction, toluene (702 mL) was added, and the resulting mixture was separated with 4% aqueous sodium bicarbonate (540 mL) to obtain a toluene layer. The obtained toluene layer was separated with 8% aqueous sodium bicarbonate (270 mL) to obtain an organic layer. Subsequently, separation with water (540 mL) was repeated twice. The resulting organic layer was concentrated under reduced pressure to obtain a solution of the title compound (IVa) in toluene (270 mL).

The title compound (IVa) was synthesized (7.29 g, yield: 84.1%) by using benzyl {3-[(2-acetamido-2-deoxy-β-D-galactopyranosyl)oxy]propyl}carbamate (IIIa) (5.0 g, 12.1 mmol) in the same manner as in (Example 1-3). ¹H-NMR (500 MHz, CD₃CN): δ 7.48-7.46 (2H, m), 7.39-7.29 (11H, m), 7.24-7.21 (1H, m), 7.11 (1H, d, J = 7.0 Hz), 6.88-6.85 (4H, m), 5.75 (1H, dd, J = 7.0, 4.5 Hz), 5.08 (1H, d, J = 12.5 Hz), 5.02 (1H, d, J = 12.5 Hz), 4.74 (1H, br s), 4.12 (1H, d, J = 8.5 Hz), 3.91 (1H, ddd, J = 9.0, 4.5, 4.5 Hz), 3.76 (6H, s), 3.68 (1H, d, J = 2.5 Hz), 3.63 (1H, ddd, J = 9.5, 8.5, 7.0 Hz), 3.55 (1H, dd, J = 7.0, 4.5 Hz), 3.46-3.37 (1H, m), 3.44 (1H, dd, J = 9.5, 2.5 Hz), 3.40 (1H, ddd, J = 9.0, 4.0, 4.0 Hz), 3.27 (1H, dd, J = 9.0, 7.0 Hz), 3.11 (1H, dd, J = 9.0, 4.5 Hz), 3.09-3.02 (1H, m), 3.02 (1H, br s), 1.94 (3H, s), 1.79-1.71 (1H, m), 1.69-1.61 (1H, m)

### (Example 1-4)

### Production of 3-aminopropyl 2-acetamido-6-O-[bis(4-methoxyphenyl)(phenyl)methyl]-2-deoxy-β-D-galactopyranoside (Ib or 6)

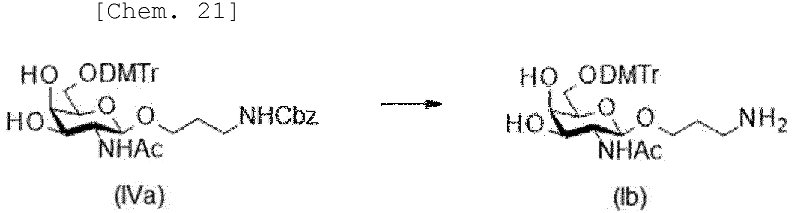

Methanol (270 mL), 5% palladium on carbon powder type PE wetted with water (5.4 g on a dry weight basis, manufactured by N.E. CHEMCAT CORPORATION), and a 25% aqueous ammonia solution (39.8 g, 655.0 mmol) were added to a solution of benzyl [3-({2-acetamido-6-O-[bis(4-methoxyphenyl)(phenyl)methyl]-2-deoxy-β-D-galactopyranosyl}oxy)propyl]carbamate (IVa) in toluene (270 mL), and the resulting mixture was stirred at 20 to 30°C for 1 hour by using an autoclave. Subsequently, the inside of the reaction system was purged with nitrogen three times, and then purged with hydrogen at 0.4 MPa (gauge pressure) three times, and the mixture was stirred at 20 to 30°C for 4 hours under a 0.3 MPa hydrogen atmosphere. After confirmation of termination of the reaction, the reaction system was purged with nitrogen three times, and the palladium on carbon was filtered off and washed with methanol (270 mL). The resulting filtrate was concentrated under reduced pressure, water (27 mL) and toluene (540 mL) were added, and the resulting mixture was concentrated under reduced pressure to obtain 540 mL of concentrate. The procedure of adding toluene (540 mL) to the obtained concentrate and concentrating the resulting mixture under reduced pressure to obtain a toluene solution (540 mL) was repeated three times. Subsequently, the precipitated solid was filtered, washed with toluene (162 mL), and dried under reduced pressure at an external temperature of 40°C to obtain the title compound (Ib or 6) (68.5 g, area ratio by liquid chromatography analysis: 99.1%, total yield for two steps: 90.1%).
¹H-NMR (500 MHz, CD₃OD): δ 7.45 (2H, d, J = 7.5 Hz), 7.35-7.31 (4H, m), 7.27 (2H, dd, J = 7.5, 7.5 Hz), 7.19 (1H, t, J = 7.5 Hz), 6.85 (4H, d, J = 8.0 Hz), 4.32 (1H, d, J = 8.5 Hz), 3.98 (1H, ddd, J = 11.5, 6.0, 5.0 Hz), 3.91 (1H, dd, J = 10.5, 8.5 Hz), 3.84 (1H, d, J = 2.5 Hz), 3.77 (6H, s), 3.62 (1H, ddd, J = 6.0, 5.0, 11.5 Hz), 3.57-3.55 (1H, m), 3.55 (1H, dd, J = 10.5, 2.5 Hz), 3.40 (1H, dd, J = 9.5, 7.0 Hz), 3.31-3.28 (1H, m), 2.82 (2H, t, J = 6.0 Hz), 1.98 (3H, s), 1.79 (2H, m)

### (Example 2)

### Production of ([12-({(2-cyanoethoxy)[di(propan-2-yl)amino]phosphanyl}oxy)-5,9,15,19-tetraoxo-10,14-dioxa-4,8,16,20-tetraazatricosane-1,23-diyl]bis(oxy)(2R,3R,4R,5R,6R)-3-acetamido-6-{[bis(4-methoxyphenyl) (phenyl)methoxy]methyl}oxane-2,4,5-triyl) tetraacetate (XIIa or 3).

### (Example 2-1)

### Production of 1,1'-{[2-(benzyloxy)propane-1,3-diyl]bis(oxycarbonyloxy)}di(pyrrolidine-2,5-dione) (Va or 7)

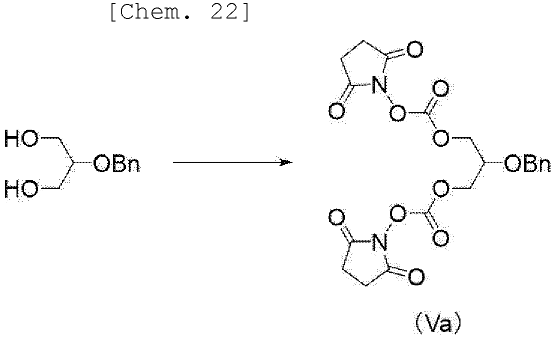

2-(Benzyloxy)propane-1,3-diol (10.0 g, 54.9 mmol) was added to acetonitrile (50 mL), and the resulting solution was temperature-regulated to 0°C. Subsequently, N,N'-disuccinimidyl carbonate (32.3 g, 126.2 mmol) and pyridine (10.9 g, 137.2 mmol) were added, and the resulting mixture was stirred at 0°C for 5 hours. After confirmation of termination of the reaction, 2-propanol (150 mL) was added to the reaction solution, and the resulting mixture was inoculated with seed crystals and stirred at 0°C for 1 hour. Subsequently, 2-propanol (100 mL) was added, and the resulting mixture was stirred at 0°C for 16 hours. The precipitated crystals were filtered, washed with 2-propanol (50 mL), and then washed with tert-butyl methyl ether (50 mL) to obtain wet crystals of the title compound (Va or 7). The obtained wet crystals were used in the next step without carrying out a drying procedure.
¹H-NMR (500 MHz, CD₃CN): δ 7.39-7.30 (5H, m), 4.65 (2H, s), 4.54 (2H, dd, J = 11.5, 4.0 Hz), 4.43 (2H, dd, J = 11.5, 5.5 Hz), 4.06 (1H, tt, J = 5.5, 4.0 Hz), 2.77 (8H, s)

### (Example 2-1-1)

### Production of 1,1'-{[2-(benzyloxy)propane-1,3-diyl]bis(oxycarbonyloxy)}di(pyrrolidine-2,5-dione) (Va or 7)

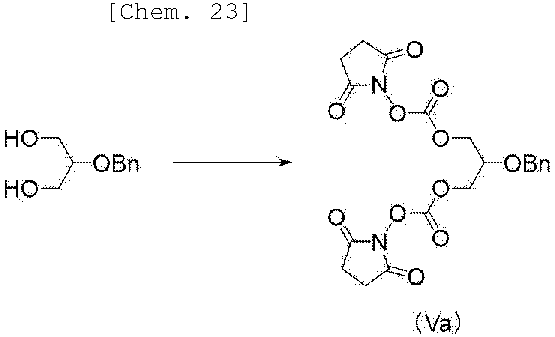

2-(Benzyloxy)propane-1,3-diol (1.0 g, 5.49 mmol) was added to acetonitrile (5 mL), N,N'-disuccinimidyl carbonate (3.2 g, 12.6 mmol) and pyridine (1.1 g, 13.7 mmol) were added, and the resulting mixture was stirred at 0°C for 4 hours. After confirmation of termination of the reaction, the mixture was allowed to stand at 0°C for 11 days to confirm the precipitation of crystals. 2-Propanol (16 mL) was added, and the resulting mixture was stirred for 1 hour at 0°C. Subsequently, 2-propanol (16 mL) was added, and the resulting mixture was stirred for 1 hour at 0°C. The precipitated crystals were filtered, washed with 2-propanol (8 mL), and then dried at an external temperature of 25°C to obtain the title compound (Va or 7) (1.40 g, area ratio by liquid chromatography analysis: 99.7%, yield: 54.9%, also used as seed crystals).
¹H-NMR (500 MHz, CD₃CN): δ 7.39-7.30 (5H, m), 4.65 (2H, s), 4.54 (2H, dd, J = 11.5, 4.0 Hz), 4.43 (2H, dd, J = 11.5, 5.5 Hz), 4.06 (1H, tt, J = 5.5, 4.0 Hz), 2.77 (8H, s)

### (Example 2-2-1)

### Production of 8-(benzyloxy)-5,11-dioxo-6,10-dioxa-4,12-diazapentadecane-1,15-dioic acid-N-benzyl-1-phenylmethanamine (1/1) (VIa or 5)

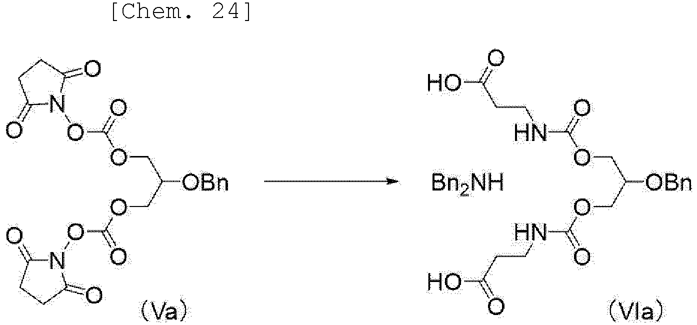

β-Alanine (14.7 g, 164.6 mmol), acetonitrile (50 mL), and triethylamine (17.2 g, 170.1 mmol) were added to water (50 mL) in this order, and the resulting mixture was stirred at 20 to 30°C. Subsequently, 1,1'-{[2-(benzyloxy)propane-1,3-diyl]bis(oxycarbonyloxy)}di(pyrrolidine-2,5-dione) (Va or 7) in wet form was added over 1.5 hours while controlling the internal temperature to 30°C or less, and the resulting mixture was stirred at 20 to 30°C for 1 hour. After confirmation of termination of the reaction, tetrahydrofuran (300 mL) and sodium chloride (50 g) were added, and the pH was adjusted to 0.1 by using 35% hydrochloric acid (28.6 g, 274.4 mmol), followed by separation to obtain an organic layer. 20% Saline (100 mL) was added to the obtained organic layer, followed by separation. The same separation procedure was carried out three more times to obtain an organic layer. The obtained organic layer was concentrated under reduced pressure to obtain a tetrahydrofuran solution (60 mL). Isopropyl acetate (200 mL) was added to the obtained tetrahydrofuran solution, and the resulting mixture was concentrated under reduced pressure to obtain 60 mL of a solution. The same procedure of concentration under reduced pressure was repeated two more times to obtain an isopropyl acetate solution (60 mL). The salt in the obtained isopropyl acetate solution was removed by filtration, and the filtrate was washed with 2-propanol (50 mL). 2-Propanol (300 mL) was added to the resulting solution, and the resulting solution was temperature-regulated to 35°C. After that, dibenzylamine (11.4 g, 57.6 mmol) was added, and the resulting mixture was inoculated with seed crystals and stirred at 35°C for 1 hour. Subsequently, the mixture was stirred at 25°C for 22 hours, and then the obtained crystals were filtered and washed with 2-propanol (50 mL) to obtain crude crystals of the title compound (VIa) in wet form (yield: 32.1 g).

Next, the obtained crude crystals were added to 2-propanol (300 mL), and the resulting mixture was temperature-regulated to 60°C to obtain a 2-propanol solution. The obtained solution was stirred at 60°C for 1 hour and then slowly cooled from 60°C to 25°C over 1 hour, and inoculated with seed crystals to confirm the precipitation of crystals. Subsequently, the slurry was stirred at 25°C for 18 hours, and then the resulting crystals were filtered, washed with 2-propanol (50 mL), and dried under reduced pressure at an external temperature of 40°C to obtain the title compound (VIa or 5) (28.2 g, area ratio by liquid chromatography analysis: 99.7%, total yield for two steps: 83.3%).
¹H-NMR (500 MHz, CD₃OD): δ 7.48-7.25 (15H, m), 4.64 (2H, s), 4.18 (2H, dd, J = 12.0, 4.5 Hz), 4.17 (4H, s), 4.10 (2H, dd, J = 12.0, 5.5 Hz), 3.86-3.78 (1H, m), 3.34 (4H, t, J = 7.0 Hz), 2.42 (4H, t, J = 7.0 Hz)

### (Example 2-2-1-1) Production of diethyl 8-(benzyloxy)-5,11-dioxo-6,10-dioxa-4,12-diazapentadecane-1,15-dioate (Va')

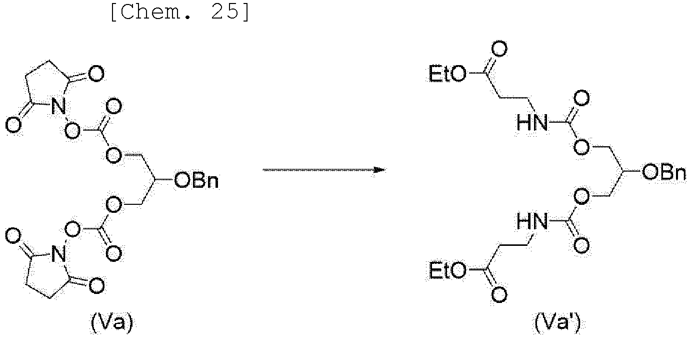

1,1'-{ [2-(Benzyloxy)propane-1,3-diyl]bis(oxycarbonyloxy)}di(pyrrolidine-2,5-dione) (Va or 7) (20.0 g, 43.0 mmol), β-alanine ethyl ester hydrochloride (16.5 g, 107.4 mmol), and triethylamine (12.2 g, 120.6 mmol) were added to tetrahydrofuran (300 mL) in this order, and the resulting mixture was stirred at 20 to 30°C for 1 hour. After confirmation of termination of the reaction, 7% aqueous sodium bicarbonate (100 mL) was added to the reaction solution, followed by separation to obtain an organic layer. 20% Saline (100 mL) was added to the obtained organic layer, followed by separation to obtain a solution of the title compound (Va') in tetrahydrofuran.

The title compound (Va') was obtained (1.58 g, yield: 78.4%) by using 1,1'-{[2-(benzyloxy)propane-1,3-diyl]bis(oxycarbonyloxy)}di(pyrrolidine-2,5-dione) (Va) (2.0 g, 4.3 mmol) in the same manner as in (Example 2-2-1-1).
¹H-NMR (500 MHz, CDCL₃): δ 7.35-7.34 (4H, d, J = 4.5 Hz), 7.31-7.27 (1H, m), 5.23 (2H, m), 4.64 (2H, s), 4.27-4.13 (8H, m), 3.82-3.77 (1H, m), 3.44 (4H, dd, J = 12.5, 5.5 Hz), 2.53 (4H, t, J = 6.0 Hz), 1.27 (6H, t, J = 6.5 Hz)

### (Example 2-2-1-2)

### Production of 8-(benzyloxy)-5,11-dioxo-6,10-dioxa-4,12-diazapentadecane-1,15-dioic acid-N-benzyl-1-phenylmethanamine (1/1) (VIa or 5)

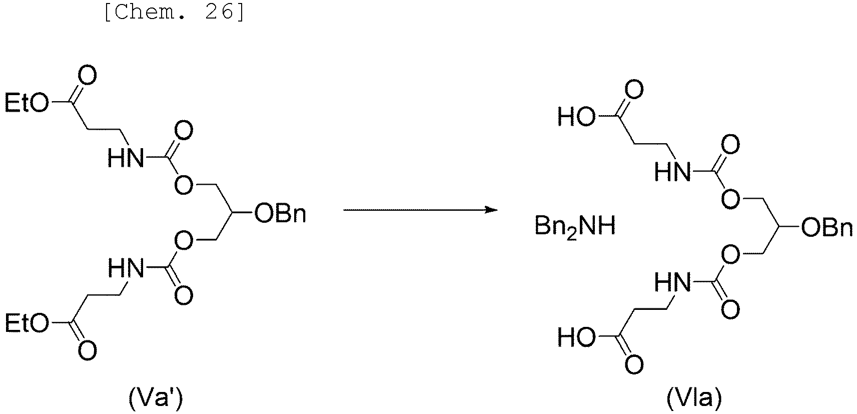

Water (100 mL) and 8 N potassium hydroxide (21.6 mL, 172.0 mmol) were added to a solution of diethyl 8-(benzyloxy)-5,11-dioxo-6,10-dioxa-4,12-diazapentadecane-1,15-dioate (Va') in tetrahydrofuran in this order, and the resulting mixture was stirred at 20 to 30°C for 3.5 hours. After confirmation of termination of the reaction, sodium chloride (10 g) was added to the reaction solution, and the pH was adjusted to 0.9 by using 35% hydrochloric acid (13.2 mL), followed by separation to obtain an organic layer. 20% Saline (100 mL) was added to the obtained organic layer, followed by separation. The resulting organic layer was concentrated under reduced pressure to obtain a tetrahydrofuran solution (100 mL). Isopropyl acetate (300 mL) and 20% saline (20 mL) were added to the obtained tetrahydrofuran solution in this order, followed by separation. The resulting organic layer was concentrated under reduced pressure to obtain an isopropyl acetate solution (100 mL). Acetonitrile (300 mL) was added to the obtained solution, and the resulting solution was temperature-regulated to 40°C. After that, dibenzylamine (2.55 g, 12.9 mmol) was added, and the resulting mixture was inoculated with seed crystals and stirred at 40°C for 4 hours. Subsequently, dibenzylamine (2.55 g, 12.9 mmol) was added, and the resulting mixture was stirred at 40°C for 19 hours. Subsequently, dibenzylamine (3.83 g, 19.4 mmol) was added, the resulting mixture was stirred at 40°C for 1 hour, and then this solution was temperature-regulated to 25°C. Subsequently, the solution was stirred at 25°C for 2 hours, and then the resulting crystals were filtered and washed with 2-propanol (50 mL) to obtain crude crystals of the title compound (VIa or 5) in wet form.

Next, the obtained crude crystals of the title compound (VIa or 5) in wet form were added to acetonitrile (300 mL), and the resulting mixture was stirred at 70°C for 1 hour and then cooled slowly from 70°C to 25°C over 2 hours to confirm the precipitation of crystals. Subsequently, the slurry was stirred at 25°C for 16 hours, and then the resulting crystals were filtered, washed with acetonitrile (100 mL), and dried under reduced pressure at an external temperature of 40°C to obtain the title compound (VIa or 5) (23.1 g, area ratio by liquid chromatography analysis: 99.98%, total yield for two steps: 88.0%).
¹H-NMR (500 MHz, CD₃OD): δ 7.48-7.25 (15H, m), 4.64 (2H, s), 4.18 (2H, dd, J = 12.0, 4.5 Hz), 4.17 (4H, s), 4.10 (2H, dd, J = 12.0, 5.5 Hz), 3.86-3.78 (1H, m), 3.34 (4H, t, J = 7.0 Hz), 2.42 (4H, t, J = 7.0 Hz)

### (Example 2-2-3)

### Production of seed crystals of 8-(benzyloxy)-5,11-dioxo-6,10-dioxa-4,12-diazapentadecane-1,15-dioic acid-N-benzyl-1-phenylmethanamine (1/1) (VIa or 5)

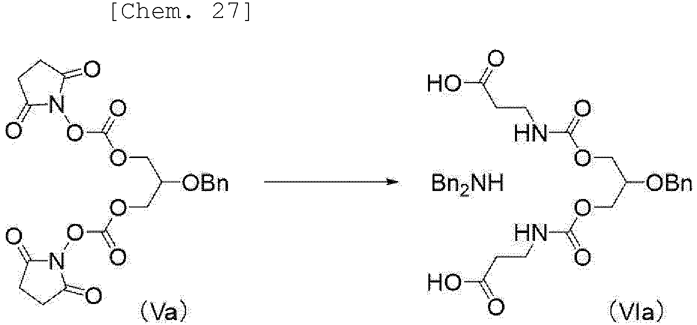

β-Alanine (19.5 g, 218.5 mmol), acetonitrile (95 mL), and triethylamine (22.9 g, 225.8 mmol) were added to water (95 mL) in this order, and the resulting mixture was stirred at 20 to 30°C. Subsequently, 1,1'-{[2-(benzyloxy)propane-1,3-diyl]bis(oxycarbonyloxy) }di(pyrrolidine-2,5-dione) (Va or 7) (38.0 g, 72.8 mmol) was added over 1 hour while controlling the internal temperature to 30°C or less, and the resulting mixture was stirred at 20 to 30°C for 31 hours. After confirmation of termination of the reaction, tetrahydrofuran (570 mL) and sodium chloride (20 g) were added, and the pH was adjusted to 0.1 by using 35% hydrochloric acid (37.9 g, 364.1 mmol), followed by separation to obtain an organic layer. 20% Saline (125 mL) was added to the obtained organic layer, followed by separation. The same separation procedure was carried out three more times. The obtained organic layer was concentrated under reduced pressure to obtain a tetrahydrofuran solution (190 mL). Cyclopentyl methyl ether (570 mL) was added to the obtained tetrahydrofuran solution, followed by separation to obtain an organic layer. The obtained organic layer was concentrated under reduced pressure to obtain 114 mL of a solution. Isopropyl acetate (570 mL) was added to the obtained organic layer, and the resulting mixture was concentrated under reduced pressure to obtain 114 mL of a solution. Isopropyl acetate (76 mL) was added to the obtained solution, subsequently acetonitrile (570 mL) was added, and the resulting solution was temperature-regulated to 40°C. After that, dibenzylamine (8.6 g, 43.7 mmol) was added, and the resulting mixture was stirred at 40°C for 19 hours. Subsequently, dibenzylamine (6.5 g, 32.8 mmol) was added, and the resulting mixture was stirred at 40°C for 2 hours. Subsequently, the mixture was stirred at 25°C for 4 hours, and then the obtained crystals were filtered and washed with acetonitrile (190 mL) to obtain seed crystals of the title compound (VIa or 5). (41.02 g, area ratio by liquid chromatography analysis: 95.8%, yield: 86.5%).
¹H-NMR (500 MHz, CD₃OD): δ 7.48-7.25 (15H, m), 4.64 (2H, s), 4.18 (2H, dd, J = 12.0, 4.5 Hz), 4.17 (4H, s), 4.10 (2H, dd, J = 12.0, 5.5 Hz), 3.86-3.78 (1H, m), 3.34 (4H, t, J = 7.0 Hz), 2.42 (4H, t, J = 7.0 Hz)

### (Example 2-3)

### Production of 2-(benzyloxy)propane-1,3-diyl bis({3-[(3-{[(2R,5R)-3-acetamido-6-{[bis(4-methoxyphenyl) (phenyl)methoxy]methyl}-4,5-dihydroxyox-2-yl]oxy}propyl)amino]-3-oxopropyl}carbamate) (VIIa or 8)

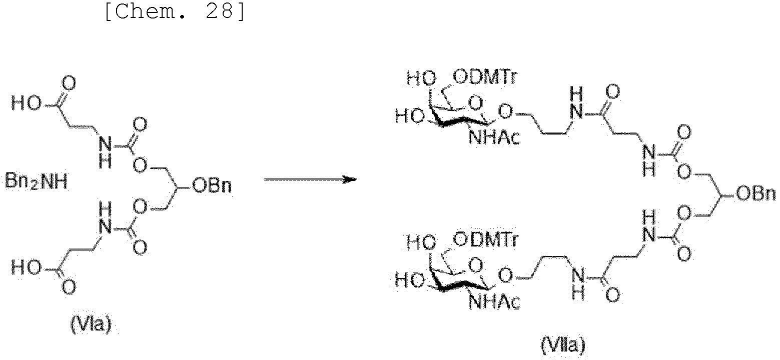

Water (200 mL), 8-(benzyloxy)-5,11-dioxo-6,10-dioxa-4,12-diazapentadecane-1,15-dioic acid-N-benzyl-1-phenylmethanamine (1/1) (VIa or 5) (20.8 g, 32.8 mmol, content: 96.2%), and triethylamine (9.96 g, 98.4 mmol) were added to dichloromethane (100 mL), and the resulting mixture was stirred at 20 to 30°C for 1 hour. After that, the mixture was separated, and the resulting aqueous layer was washed twice with dichloromethane (60 mL). Tetrahydrofuran (600 mL), 1-hydroxybenzotriazole monohydrate (10.6 g, 68.9 mmol), 3-aminopropyl 2-acetamido-6-o-[bis(4-methoxyphenyl)(phenyl)methyl]-2-deoxy-β-D-galactopyranoside (Ib) (37.9 g, 62.3 mmol), and 1-(3-dimethylaminopropyl)-3-ethylcarbodiimide hydrochloride (15.7 g, 82.0 mmol) were added to the resulting aqueous layer in this order, and the resulting mixture was stirred at 20 to 30°C for 5 hours. After that, 3-aminopropyl 2-acetamido-6-O-[bis(4-methoxyphenyl)(phenyl)methyl]-2-deoxy-β-D-galactopyranoside (Ib) (2.53 g, 4.16 mmol) and 1-(3-dimethylaminopropyl)-3-ethylcarbodiimide hydrochloride (1.67 g, 8.71 mmol) were added, and the resulting mixture was stirred for 11 hours; next, 3-aminopropyl 2-acetamido-6-O-[bis(4-methoxyphenyl)(phenyl)methyl]-2-deoxy-β-D-galactopyranoside (Ib) (178.0 mg, 293 µmol) and 1-(3-dimethylaminopropyl)-3-ethylcarbodiimide hydrochloride (117 mg, 610 µmol) were added, and the resulting mixture was stirred for 7 hours; and furthermore, 3-aminopropyl 2-acetamido-6-O-[bis(4-methoxyphenyl)(phenyl)methyl]-2-deoxy-β-D-galactopyranoside (Ib) (51.0 mg, 84 µmol) and 1-(3-dimethylaminopropyl)-3-ethylcarbodiimide hydrochloride (34.0 mg, 177 µmol) were added, and the resulting mixture was stirred for 23 hours. After confirmation of termination of the reaction, the mixture was washed with 20% saline (200 mL), ethyl acetate (600 mL) was added, and separation with 8% aqueous sodium bicarbonate (400 mL) was repeated twice. The resulting organic layer was concentrated under reduced pressure to obtain 300 mL of a solution. Subsequently, the procedure of adding tetrahydrofuran (300 mL) and ethyl acetate (300 mL) and concentrating the resulting mixture under reduced pressure to obtain 300 mL of a solution was repeated twice. Tetrahydrofuran (1000 mL) was added to the resulting solution, and the resulting mixture was concentrated under reduced pressure to obtain 200 mL of a solution. Tetrahydrofuran (1000 mL) was added to the obtained solution, and the resulting mixture was concentrated under reduced pressure to obtain 244 mL of a solution. Tetrahydrofuran (56 mL) was added to the obtained solution to obtain a solution of the title compound (VIIa or 8) in ethyl acetate/tetrahydrofuran (300 mL).

The title compound (VIIa or 8) was synthesized by using 8-(benzyloxy)-5,11-dioxo-6,10-dioxa-4,12-diazapentadecane-1,15-dioic acid-N-benzyl-1-phenylmethanamine (1/1) (VIa or 5) (211.2 mg, 0.4 mmol) in the same manner as in (Example 2-3), and isolated by silica gel column purification (186.4 mg, yield: 96.7%). ¹H-NMR (500 MHz, CD₃OD): δ 7.78 (2H, t, J = 5.5 Hz), 7.68 (2H, d, J = 9.0 Hz, 7.41 (4H, d, J = 7.5 Hz), 7.32-7.25 (19H, m), 7.21 (2H, t, J = 7.5 Hz), 6.88 (8H, d, J = 8.5 Hz), 4.66 (2H, d, J = 6.0 Hz), 4.57 (2H, s), 4.54 (2H, d, J = 4.0 Hz), 4.26 (2H, d, J = 8.0 Hz), 4.10 (2H, dd, J = 11.5, 4.0 Hz), 3.99 (2H, dd, J = 11.5, 6.0 Hz), 3.76-3.68 (5H, m, ), 3.73 (12H, s), 3.63 (2H, dd, J = 3.5, 3.5 Hz), 3.54 (2H, t, J = 6.5 Hz), 3.47-3.40 (4H, m), 3.19-3.10 (8H, m), 3.06-2.99 (4H, m), 2.23 (4H, t, J = 7.5 Hz), 1.82 (6H, s), 1.66-1.57 (4H, m)

### (Example 2-4)

### Production of ([12-(benzyloxy)-5,9,15,19-tetraoxo-10,14-dioxa-4,8,16,20-tetraazatricosane-1,23-diyl]bis(oxy) (2R,5R)-3-acetamido-6-{[bis(4-methoxyphenyl) (phenyl)methoxy]methyl}oxane-2,4,5-triyl) tetraacetate (VIIIa or 2)

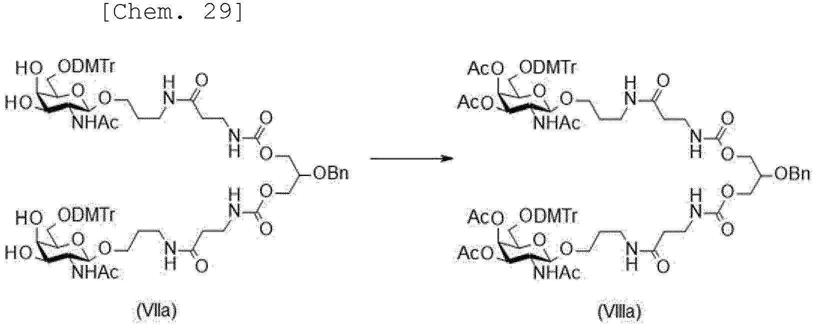

Tetrahydrofuran (10 mL) and triethylamine (29.9 g, 295.0 mmol) were added to a solution of 2-(benzyloxy)propane-1,3-diyl bis({3-[(3-{[(2R,5R)-3-acetamido-6-{ [bis(4-methoxyphenyl) (phenyl)methoxy]methyl}-4,5-dihydroxyox-2-yl]oxy}propyl)amino]-3-oxopropyl}carbamate) (VIIa or 8) in ethyl acetate/tetrahydrofuran (300 mL), and the resulting mixture was stirred for 10 minutes. Subsequently, 4-dimethylaminopyridine (1.20 g, 9.8 mmol) and acetic anhydride (26.8 g, 263.0 mmol) were added in this order, and the resulting mixture was stirred at 20 to 30°C for 8 hours. After confirmation of termination of the reaction, ethyl acetate (1000 mL) and 8% aqueous sodium bicarbonate (500 mL) were added, and the resulting mixture was stirred at 20 to 30°C for 22 hours and then separated. The resulting organic layer was washed with water (300 mL), a 5% aqueous potassium dihydrogen phosphate solution (300 mL), 8% aqueous sodium bicarbonate (300 mL), and water (300 mL) in this order. The resulting organic layer was concentrated under reduced pressure to obtain an ethyl acetate solution (400 mL). N-Methyl-2-pyrrolidone (200 mL) and ethyl acetate (400 mL) were added to the obtained ethyl acetate solution, and the resulting mixture was concentrated under reduced pressure to obtain an ethyl acetate/N-methyl-2-pyrrolidone solution (600 mL). Subsequently, the procedure of adding ethyl acetate (400 mL) to the obtained solution and concentrating the resulting mixture under reduced pressure to obtain an ethyl acetate/N-methyl-2-pyrrolidone solution (600 mL) was repeated three times to obtain a solution of the title compound (VIIIa) in ethyl acetate/N-methyl-2-pyrrolidone (600 mL).

The title compound (VIIIa or 2) was synthesized by using 2-(benzyloxy)propane-1,3-diyl bis({3-[(3-{[(2R,5R)-3-acetamido-6-{ [bis(4-methoxyphenyl) (phenyl)methoxy]methyl}-4,5-dihydroxyox-2-yl]oxy}propyl)amino]-3-oxopropyl}carbamate) (VIIa) (33.0 g, 21.46 mmol) in the same manner as in (Example 2-4), and isolated by silica gel column purification (38.20 g, yield: 104.4%).
¹H-NMR (500 MHz, CD₃OD): δ 7.82 (2H, d, J = 9.5 Hz), 7.75 (2H, t, J = 5.5 Hz), 7.34-7.17 (25H, m), 6.91-6.86 (8H, m), 5.40 (2H, d, J = 3.5 Hz), 4.99 (2H, dd, J = 11.5, 3.5 Hz), 4.57 (2H, s), 4.45 (2H, d, J = 8.5 Hz), 4.10 (2H, dd, J = 11.5, 4.0 Hz), 4.08-4.05 (2H, m), 3.99 (2H, dd, J = 11.5, 5.5 Hz), 7.62 (2H, ddd, J = 11.5, 9.5, 8.5 Hz), 3.76-3.70 (1H, m), 3.74 (12H, s), 3.66 (2H, ddd, J = 10.0, 6.5, 6.5 Hz), 3.37 (2H, ddd, J = 10.0, 6.5, 6.5 Hz), 3.17-3.11 (6H, m), 3.08-3.00 (2H, m), 3.02-2.94 (2H, m), 2.80 (2H, t, J = 8.5 Hz), 2.22 (4H, t, J = 7.0 Hz), 1.89 (6H, s), 1.86 (6H, s), 1.77 (6H, s), 1.56 (4H, dddd, J = 7.0, 7.0, 6.5, 6.5 Hz)

### (Example 2-5)

### Production of [(12-hydroxy-5,9,15,19-tetraoxo-10,14-dioxa-4,8,16,20-tetraazatricosane-1,23-diyl)bis(oxy) (2R,5R)-3-acetamido-6-{ [bis(4-methoxyphenyl) (phenyl)methoxy]methyl}oxane-2,4,5-triyl] tetraacetate (IXa or 1)

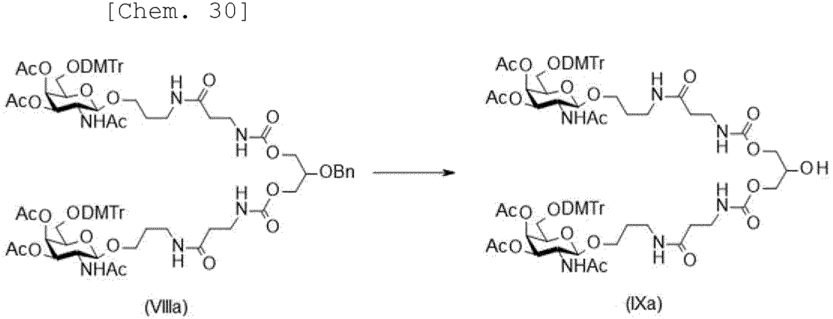

A 5% palladium on carbon powder (type PE wetted with water) (2.1 g, on a dry weight basis, manufactured by N.E. CHEMCAT CORPORATION) was added to a solution of ([12-(benzyloxy)-5,9,15,19-tetraoxo-10,14-dioxa-4,8,16,20-tetraazatricosane-1,23-diyl]bis(oxy) (2R,5R)-3-acetamido-6-{ [bis(4-methoxyphenyl) (phenyl)methoxy]methyl}oxane-2,4,5-triyl) tetraacetate (VIIIa or 2) in ethyl acetate/N-methyl-2-pyrrolidone (600 mL), and the inside of the system was purged with nitrogen three times by using an autoclave, and then purged with 0.1 MPa hydrogen three times. After that, the mixture was stirred at 20 to 30°C for 7 hours under a 0.1 MPa hydrogen atmosphere. After confirmation of termination of the reaction, the reaction system was purged with nitrogen three times, and the palladium on carbon was filtered off and washed with ethyl acetate (200 mL). Ethyl acetate (600 mL) and 2.5% saline (600 mL) were added to the resulting solution, followed by separation. The separation of the resulting organic layer with a mixed solution of 2.5% saline (600 mL) and N-methyl-2-pyrrolidone (200 mL) was repeated three times to obtain an organic layer. The separation of the obtained organic layer with 2.5% saline (600 mL) was repeated three times. After that, the procedure of adding ethyl acetate (600 mL) and concentrating the resulting mixture under reduced pressure to obtain an ethyl acetate solution (800 mL) was repeated twice. The obtained ethyl acetate solution (800 mL) was purified through a column (CHROMATOREX DIOL MB100-40/75 800 g, ethyl acetate (16 L) was passed through, and then a 3% methanol/ethyl acetate solution (24 L) was passed through), and the recovered fraction was concentrated under reduced pressure to obtain a solution of the title compound (IXa or 1) in ethyl acetate (800 mL) (quantitative value by liquid chromatography analysis: 40.0 g, area ratio: 99.7%, total yield for three steps: 75.4%).
¹H-NMR (500 MHz, CD₃CN): δ 7.40-7.38 (4H, m), 7.31-7.21 (14H, m), 6.88-6.84 (8H, m), 7.21-6.66 (2H, br), 6.59 (2H, br s), 5.91-5.87 (2H, br), 5.43 (2H, d, J = 3.5 Hz), 5.01 (2H, dd, J = 11.5, 3.5 Hz), 4.45 (2H, d, J = 8.5 Hz), 4.10-3.88 (8H, m), 3.85-3.79 (1H, m), 3.81 (2H, ddd, J = 10.5, 5.5, 5.0 Hz), 3.76 (12H, s), 3.46 (2H, ddd, J = 10.5, 7.5, 5.0 Hz), 3.34-3.27 (6H, m), 3.23 (2H, dd, J = 9.0, 5.5 Hz), 3.07 (2H, ddd, J = 11.5, 11.5, 5.5 Hz), 2.90 (2H, dd, J = 9.0, 8.5 Hz), 2.34-2.25 (4H, m), 1.91 (6H, s), 1.88 (6H, s), 1.86 (6H, s), 1.70-1.59 (4H, m)

### (Example 2-5-1)

### Benzyl group deprotection study 1

In the reaction for obtaining compound (IXa or 1) from compound (VIIIa or 2), reaction solvents were screened.

Under the same reaction conditions as in Example 2-5, reactions were carried out by using a 10% palladium on carbon powder (type AD wetted with water) (manufactured by Kawaken Fine Chemicals Co., Ltd.) and using the organic solvents shown in Table 2 as reaction solvents, respectively. Results thereof are shown in Table 2. In the following reaction equation and table, compound (Xa) is an impurity resulting from the loss of one DMTr group from compound (IXa or 1) due to an excessive reduction reaction, and compound (XIa) is an impurity derived from the lost protecting group (DMTr group).

### HPLC analysis conditions

Detection: 210 nm
Column: Xbridge C18 (4.6 mm ID × 150 mm, 3.5 µm)
Column temperature: 40°C
Mobile phases: A: 10 mM phosphate buffer, B: acetonitrile
Gradient conditions: B (conc%) 55 (0-15 min), 55-75 (15-30 min), 75-55 (30-30.01 min), 55 (30.01-40 min)
Flow rate: 1.0 mL/min
Injection volume: 5 µL

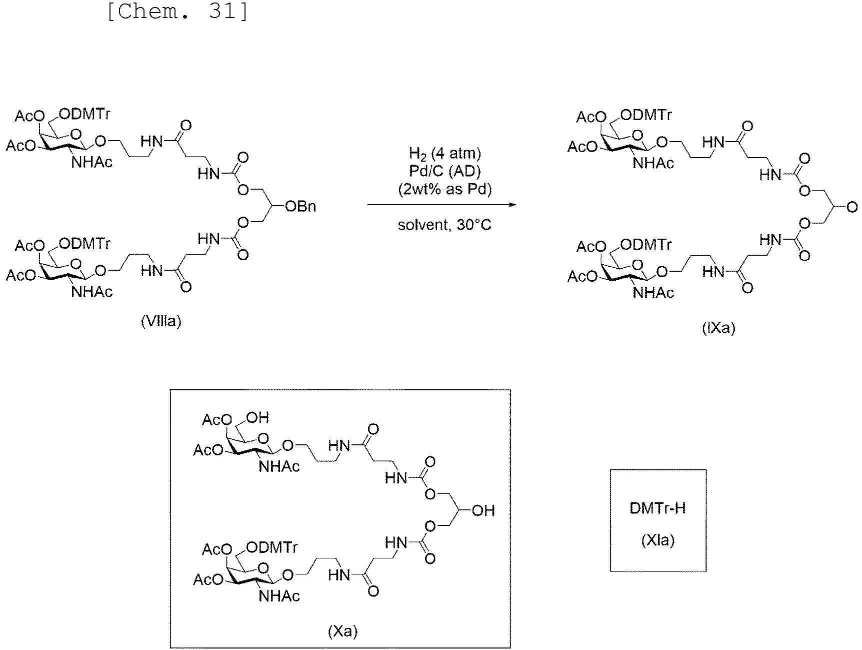

**[Table 2]**

| Exp. No. | Catalyst* (2wt% as Pd) | H₂ (bar) | solvent | HPLC p.a.%, retention time (min) | | | |
|---|---|---|---|---|---|---|---|
| | | | | VIIIa | IXa | Xa | XIa |
| 1 | 10%Pd/C | 4 | MeOH | 13.91 | 45.91 | 2.90 | 14.38 |
| 2 | 10%Pd/C | 4 | IPA | 1.59 | 85.78 | 0.31 | 2.88 |
| 3 | 10%Pd/C | 4 | EtOAc | 12.60 | 59.99 | 2.85 | 17.13 |
| 4 | 10%Pd/C | 4 | 2-MeTHF | 12.27 | 69.43 | 1.81 | 7.84 |
| 5 | 10%Pd/C | 4 | DMAc | 27.55 | 63.03 | 0.14 | 0.72 |
| 6 | 10%Pd/C | 4 | NMP | 29.59 | 62.00 | 0.13 | 0.60 |

### (Example 2-5-2)

### Benzyl group deprotection study 2

In order to facilitate separation after completion of the reaction and reduce loss of product into the aqueous layer, a study using a mixed solvent of ethyl acetate and any of various polar solvents was carried out. The present step is a continuation step, and if the reaction could be carried out in a mixed solvent with ethyl acetate, which is the post-treatment solvent in the preceding step, the step would be a simpler step. Therefore, when the present reaction was carried out in a mixed solvent, a study was carried out to confirm the optimal mixed system of ethyl acetate and any of various polar solvents. A 5% palladium on carbon powder (type PE wetted with water) (manufactured by N.E. CHEMCAT CORPORATION) was used under the same reaction conditions as in Example 2-5. Results thereof are shown in Table 3.

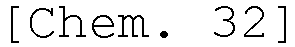

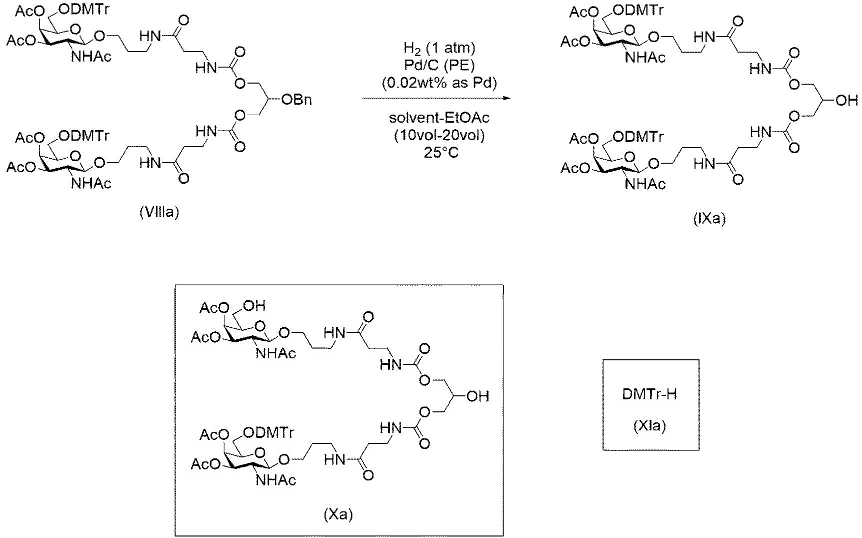

**[Table 3]**

| Exp. No. | solvent | HPLC p.a.%, retention time (min) | | | |
|---|---|---|---|---|---|
| | | VIIIa | IXa | Xa | XIa |
| I | NMP | Not Detected | 90.0 | 2.7 | 3.3 |
| 2 | DMAc | Not Detected | 85.3 | 5.2 | 6.0 |
| 3 | DMI | Not Detected | 83.3 | 6.2 | 7.0 |
| 4 | DMSO | Not Detected | 6.7 | Not Detected | Not Detected |

### (Example 2-5-3)

### Benzyl group deprotection study 3

A 5% palladium on carbon powder (type PE wetted with water) (N.E. CHEMCAT CORPORATION) was used under the same reaction conditions as in Example 2-5 to study the influence of the solvent composition ratio of N-methyl-2-pyrrolidone and ethyl acetate on the reaction yield. Results thereof are shown in Table 4.

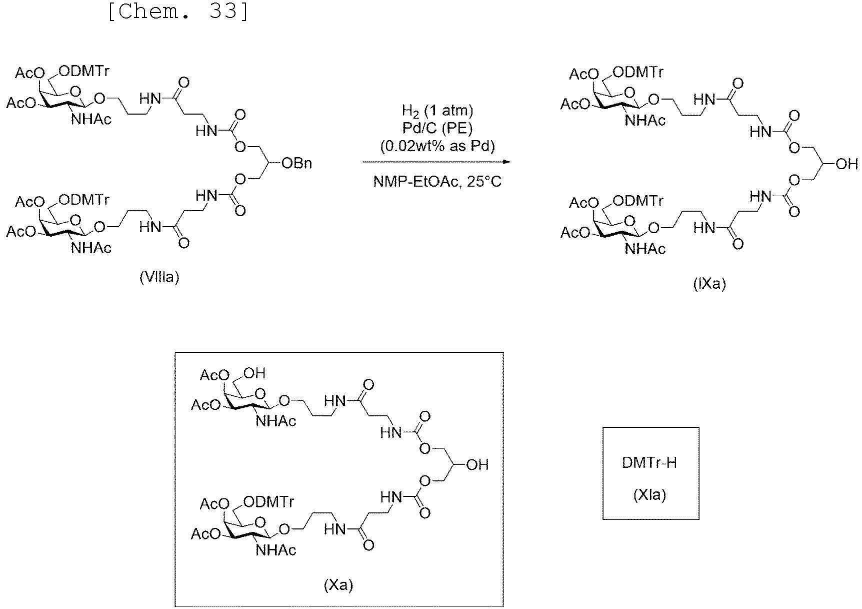

### HPLC analysis conditions

Detection: 210 nm
Column: Xbridge C18 (4.6 mm ID × 150 mm, 3.5 µm)
Column temperature: 40°C
Mobile phases: A: 10 mM ammonium acetate buffer, B: acetonitrile
Gradient conditions: B (conc%) 5 (0-3 min), 5-95 (3-30 min), 95 (30-33 min), 95-5 (33-33.1 min), 5 (33.1-42 min)
Flow rate: 1.0 mL/min
Injection volume: 5 µL

**[Table 4]**

| Exp. No. | EtOAc | NMP | HPLC p.a.%, retention time (min) | | | |
|---|---|---|---|---|---|---|
| | | | VIIIa | IXa | Xa | XIa |
| 1 | 20 | 10 | Not Detected | 90.0 | 2.7 | 3.3 |
| 2 | 25 | 5 | 0.02 | 85.4 | 5.1 | 5.8 |
| 3 | 30 | 1 | 0.6 | 54.0 | 15.0 | 26.7 |

### (Example 2-5-4)

### Benzyl group deprotection study 4

A 5% palladium on carbon powder (type PE wetted with water) (manufactured by N.E. CHEMCAT CORPORATION) was used under the same reaction conditions as in Example 2-5 to study the amount of the catalyst. Results thereof are shown in Table 5.

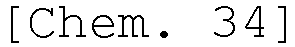

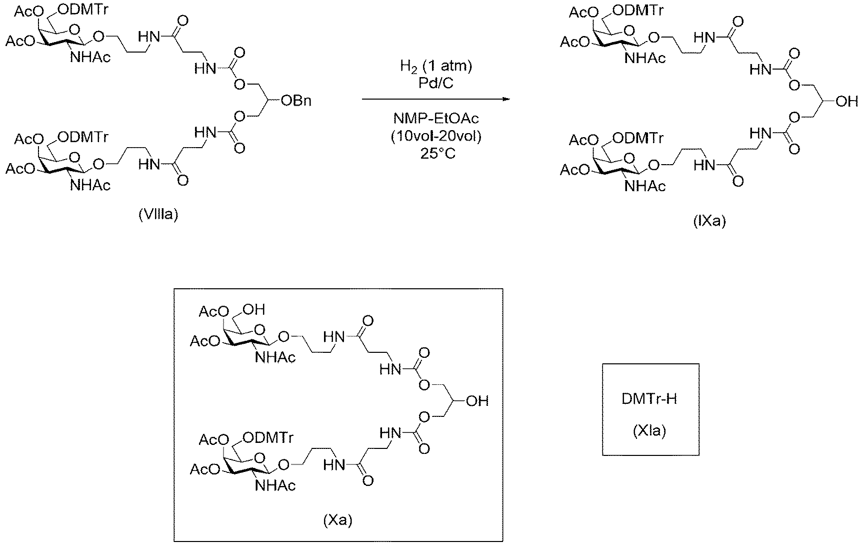

### HPLC analysis conditions

Detection: 210 nm
Column: Xbridge C18 (4.6 mm ID × 150 mm, 3.5 µm)
Column temperature: 40°C
Mobile phases: A: 10 mM phosphate buffer, B: acetonitrile Gradient conditions: B (conc%) 55 (0-15 min), 55-75 (15-30 min), 75-55 (30-30.01 min), 55 (30.01-40 min)
Flow rate: 1.0 mL/min
Injection volume: 5 µL

**[Table 5]**

| Exp. No. | Catalyst | H₂ (bar) | temp. (°C) | HPLC p.a.%, retention time (min) | | | |
|---|---|---|---|---|---|---|---|
| | | | | VIIIa | IXa | Xa | XIa |
| 1 | 20%Pd(OH)₂/C (0.1wt% as Pd) | 1 | 30 | Not Detected | 84.42 | 3.35 | 4.87 |
| 2 | 20%Pd(OH)₂/C (0.07wt% as Pd) | 1 | 30 | 22.99 | 63.80 | 0.97 | 1.87 |
| 3 | 5%Pd/C* (0.03wt% as Pd) | 1 | 30 | Not Detected | 78.56 | 7.40 | 7.82 |
| 4 | 5%Pd/C* (0.01wt% as Pd) | 1 | 30 | Not Detected | 85.76 | 3.63 | 4.03 |

### (Example 2-6)

### Production of ([12-({(2-cyanoethoxy)[di(propan-2-yl)amino]phosphanyl}oxy)-5,9,15,19-tetraoxo-10,14-dioxa-4,8,16,20-tetraazatricosane-1,23-diyl]bis(oxy)(2R,3R,4R,5R,6R)-3-acetamido-6-{[bis(4-methoxyphenyl) (phenyl)methoxy]methyl}oxane-2,4,5-triyl) tetraacetate (XIIa or 3)

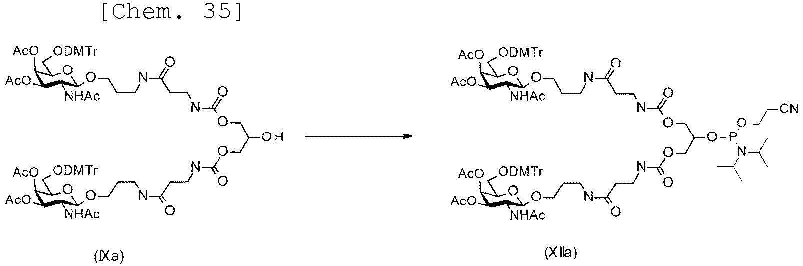

A solution of [(12-hydroxy-5,9,15,19-tetraoxo-10,14-dioxa-4,8,16,20-tetraazatricosane-1,23-diyl)bis(oxy)(2R,5R)-3-acetamido-6-{[bis(4-methoxyphenyl) (phenyl)methoxy]methyl}oxane-2,4,5-triyl] tetraacetate (IXa or 1) in ethyl acetate (600 mL, quantitative value by liquid chromatography: 30.0 g, 18.6 mmol) was concentrated under reduced pressure to obtain an ethyl acetate solution (90 mL). Ethyl acetate (900 mL) was added to the obtained solution, and the resulting mixture was concentrated under reduced pressure to 90 mL. Dichloromethane (300 mL) and molecular sieve 4A (15.0 g) were added to the resulting solution, and the resulting mixture was stirred at 20 to 30°C for 45 minutes. After that, the mixture was cooled to 0°C, 2-cyanoethyl N,N,N',N'-tetraisopropylphosphordiamidite (7.27 g, 24.1 mmol) and 4,5-dicyanoimidazole (1.10 g, 9.31 mmol) were added, and the resulting mixture was stirred at 2 to 4°C for 16 hours. After confirmation of termination of the reaction, the reaction solution was passed through a pad of CHROMATOREX (registered trademark) PEI MB100-40/75 (60.0 g), and the pad was washed with dichloromethane (180 mL). The resulting solution was filtered and washed with dichloromethane (90 mL). The resulting solution was concentrated under reduced pressure to obtain a dichloromethane/ethyl acetate solution (180 mL). The procedure of adding ethyl acetate (600 mL) to this solution and concentrating the resulting mixture under reduced pressure was repeated twice to obtain an ethyl acetate solution (300 mL). tert-Butyl methyl ether (270 mL) was added to the obtained ethyl acetate solution to obtain an ethyl acetate/tert-butyl methyl ether solution as solution A (570 mL).

In a fresh container, tert-butyl methyl ether (300 mL) and n-heptane (900 mL) were added and cooled to 4 to 5°C, and then solution A (570 mL) was poured therein, followed by rinsing with a 50% ethyl acetate/tert-butyl methyl ether solution (30 mL) and stirring at 2 to 8°C for 2.5 hours. The resulting slurry was filtered, washed with a 25% tert-butyl methyl ether/n-heptane solution (180 mL) pre-cooled to 0°C and n-heptane (300 mL) pre-cooled to 0°C in this order, and dried under vacuum at an external temperature of 40°C to obtain a powder of the title compound (XIIa or 3) (34.3 g, area ratio by liquid chromatography analysis: 98.4%, yield: 101.7%).
¹H-NMR (500 MHz, CD₃CN): δ 7.39 (4H, d, J = 8.0 Hz), 7.31-7.21 (14H, m), 6.87-6.83 (8H, m), 6.71-6.62 (2H, br), 6.57 (2H, brs), 5.97-5.83 (2H, br), 5.42 (2H, d, J = 3.5 Hz), 5.00 (2H, dd, J = 11.5 Hz), 4.44 (2H, d, J = 8.5 Hz), 4.20-3.97 (5H, m), 3.95-3.88 (4H, m), 3.83-3.70 (16H, m), 3.64-3.54 (2H, qq, J = 7.0, 6.5 Hz), 3.46 (2H, ddd, J = 10.0, 8.0, 5.0 Hz), 3.33-3.25 (6H, m), 3.23 (2H, dd, J = 9.0, 6.0 Hz), 3.10-3.04 (2H, m), 2.90 (2H, dd, J = 9.0, 8.0 Hz), 2.64 (1H, dd, J = 7.0, 5.5 Hz), 2.63 (1H, dd, J = 7.0, 5.5 Hz), 2.36-2.23 (4H, m), 1.91 (6H, s), 1.88 (6H, s), 1.86 (6H, s), 1.70-1.58 (4H, m), 1.15 (6H, d, J = 7.0 Hz), 1.14 (6H, d, J = 6.5 Hz)

Compound (XIIa or 3) can be converted into an N-acetyl-D-galactosamine ligand-oligonucleotide conjugate, for example, by using the method disclosed in International Publication No. WO 2019/172286, International Publication No. WO 2021/049504, or the like.

### Industrial Applicability

By using the present invention, it is possible to provide a novel method for producing a biantennary N-acetyl-D-galactosamine ligand-oligonucleotide conjugate, which is useful as a pharmaceutical.

## Claims

1. A method for producing the compound represented by formula (1): 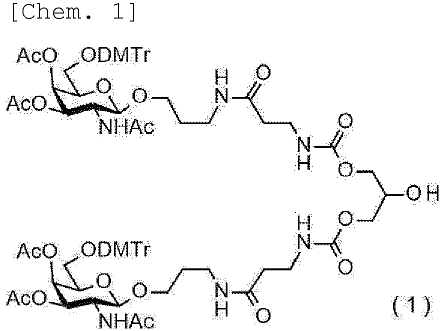
wherein Ac represents an acetyl group, and DMTr represents a 4,4'-dimethoxytrityl group,
the method comprising a step of reacting the compound represented by formula (2): 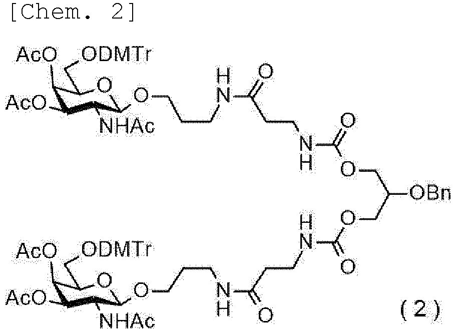
wherein Ac represents an acetyl group, DMTr represents a 4,4'-dimethoxytrityl group, and Bn represents a benzyl group,
with a palladium catalyst in the presence of a reducing agent in a reaction solvent.

2. The production method according to claim 1, wherein the palladium catalyst is palladium on carbon or palladium hydroxide on carbon.

3. The production method according to claim 1 or 2, wherein the reaction solvent is one or more solvents selected from an alcohol-based solvent, an ester-based solvent, an ether-based solvent, and an amide-based solvent.

4. The production method according to claim 1 or 2, wherein the reaction solvent is one or more solvents selected from 2-propanol, dimethylacetamide, N-methyl-2-pyrrolidone, and 1,3-dimethyl-2-imidazolidinone.

5. The production method according to claim 1 or 2, wherein the reaction solvent is a mixed solvent of ethyl acetate and one or more solvents selected from dimethylacetamide, N-methyl-2-pyrrolidone, and 1,3-dimethyl-2-imidazolidinone.

6. A method for producing the compound represented by formula (3): 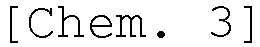 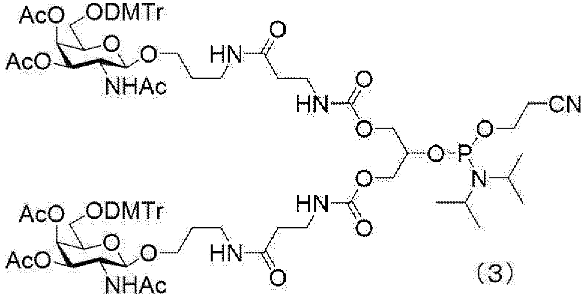
wherein Ac represents an acetyl group, and DMTr represents a 4,4'-dimethoxytrityl group,
or a salt thereof,
the method comprising a step of reacting the compound represented by formula (1) obtained by the production method according to any one of claims 1 to 5 with an amidite reagent.

7. A method for producing a conjugate consisting of an oligonucleotide and a biantennary N-acetyl-D-galactosamine unit, the biantennary N-acetyl-D-galactosamine unit being bonded to a 5' end or a 3' end of the oligonucleotide, the conjugate being represented by formula (4): 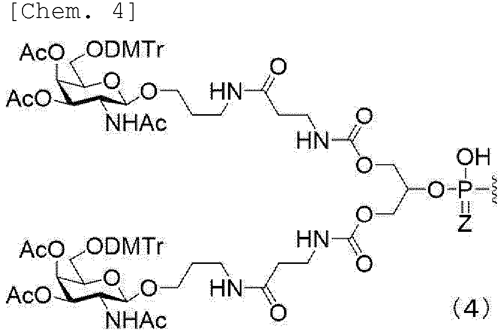
wherein Ac represents an acetyl group, Z represents an oxygen atom or a sulfur atom, and the bond at the right side of the structural formula represents a bond to the oligonucleotide,
the method comprising a step of reacting the compound represented by formula (3) obtained by the production method according to claim 6 with the oligonucleotide.

8. A method for producing the compound represented by formula (2) according to claim 1, comprising: a step of bonding the compound represented by formula (5) : 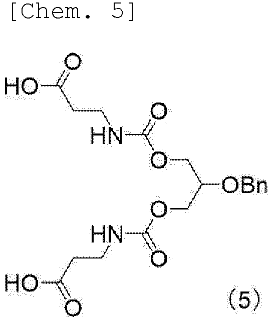
wherein Bn represents a benzyl group,
or a salt thereof, and
the compound represented by formula (6): 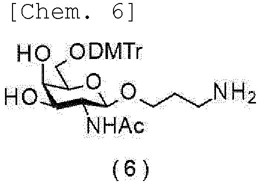
wherein Ac represents an acetyl group, and DMTr represents a 4,4'-dimethoxytrityl group,
by using a condensing agent, and
a step of acetylating a hydroxyl group on the pyran ring by using an acetylation reagent.

9. A method for producing the compound represented by formula (5) according to claim 8 or a salt thereof, comprising:
a step of reacting the compound represented by formula (7) : 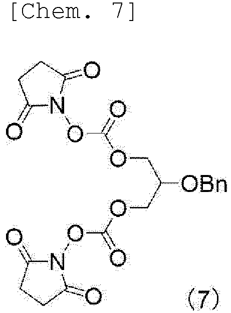
wherein Bn represents a benzyl group,
with β-alanine or a salt thereof, or
a step of reacting the compound represented by formula (7) with a β-alanine alkyl ester or a salt thereof, wherein
when the method comprises a step using a β-alanine alkyl ester or a salt thereof, the method further comprises a hydrolysis step.

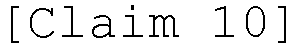

10. A method for producing the compound represented by formula (7) according to claim 9, comprising:
a step of reacting 2-(benzyloxy)propane-1,3-diol with N,N'-disuccinimidyl carbonate in the presence of a base.

11. A compound represented by formula (2): 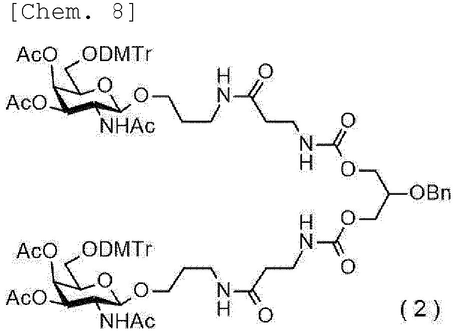 wherein Ac represents an acetyl group, DMTr represents a 4,4'-dimethoxytrityl group, and Bn represents a benzyl group.

12. A compound represented by formula (8): 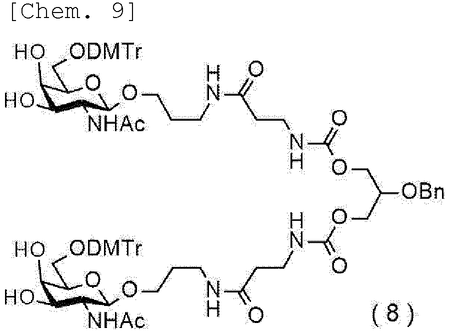 wherein Ac represents an acetyl group, DMTr represents a 4,4'-dimethoxytrityl group, and Bn represents a benzyl group.

13. A compound represented by formula (5): 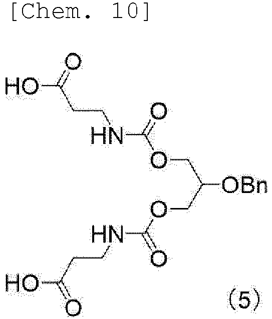
wherein Bn represents a benzyl group,
or a salt thereof.

14. A compound represented by formula (7): 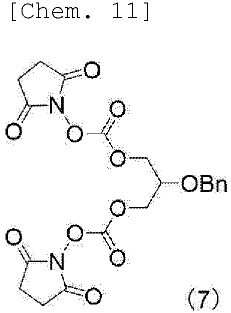 wherein Bn represents a benzyl group.

15. A compound represented by formula (6): 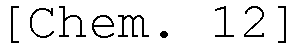 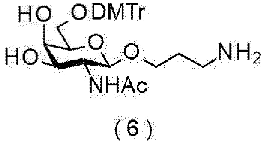
wherein Ac represents an acetyl group, and DMTr represents a 4,4'-dimethoxytrityl group,
or a salt thereof.
